# EUROPÄISCHE PATENTSCHRIFT

(11) **EP 2 323 645 B1**
(45) Veröffentlichungstag und Bekanntmachung des Hinweises auf die Patenterteilung: **10.09.2014**
(21) Anmeldenummer: 09777892.2
(22) Anmeldetag: 14.08.2009
(51) Int. Cl.: A61K 31/167, A61P 25/00, A61P 25/06

(54) **VERWENDUNG NIEDRIG-DOSIERTER LOKALANÄSTHETIKA ODER DERIVATE DAVON ZUR THERAPIE CHRONISCHER SCHMERZEN, INSBESONDERE MIGRÄNE**
USE OF LOW-DOSE LOCAL ANAESTHETIC OR DERIVATIVES THEREOF FOR THERAPY OF CHRONIC PAIN, ESPECIALLY MIGRAINE
UTILISATION À FAIBLE DOSE D'ANESTHÉSIQUES LOCAUX OU DE LEURS DÉRIVÉS POUR LA THÉRAPIE DE DOULEURS CHRONIQUES, EN PARTICULIER LA MIGRAINE

(30) Priorität: 14.08.2008 DE 102008037682
(43) Veröffentlichungstag der Anmeldung: 25.05.2011
(73) Patentinhaber: Strackharn, Klaus, 76530 Baden-Baden (DE); Weihe, Eberhard, 35037 Marburg (DE)
(72) Erfinder: Strackharn, Klaus, 76530 Baden-Baden (DE); Weihe, Eberhard, 35037 Marburg (DE)
(74) Vertreter: Graf von Stosch, Andreas
(86) Internationale Anmeldenummer: PCT/EP2009/005917
(87) Internationale Veröffentlichungsnummer: WO 2010/017996

(56) Entgegenhaltungen:
- WO-A-99/03473
- BROFELDT B T ET AL: "Pericranial injection of local anesthetics for the management of resistant headaches" ACADEMIC EMERGENCY MEDICINE, HANLEY AND BELFUS, PHILADELPHIA, PA, US, [Online] Bd. 5, Nr. 12, 1. Dezember 1998 (1998-12-01), Seiten 1224-1229, XP009125869 ISSN: 1069-6563 Gefunden im Internet: URL:http://www3.interscience.wiley.com/cgi -bin/issn?DESCRIPTOR=PRINTISSN& VALUE=1069-6563>
- ASHKENAZI AVI ET AL: "Greater occipital nerve block for chronic daily headache using local anesthetics with or without corticosteroids - A randomized single-blind study" NEUROLOGY, LIPPINCOTT WILLIAMS & WILKINS, PHILADELPHIA, US, Bd. 66, Nr. 5, Suppl. 2, 1. März 2006 (2006-03-01), Seite A223, XP009125878 ISSN: 0028-3878
- DOI KATSUSHI ET AL: "Intercostal nerve block with 5% tetracaine for chronic pain syndromes" JOURNAL OF CLINICAL ANESTHESIA, BUTTERWORTH PUBLISHERS, STONEHAM, GB, [Online] Bd. 14, Nr. 1, 1. Februar 2002 (2002-02-01), Seiten 39-41, XP009125870 ISSN: 0952-8180 Gefunden im Internet: URL:http://www.sciencedirect.com/science/j ournal/09528180>
- GROSS D: "KONTRALATERALE LOKALANAESTHESIE BEI AKUTEN UND CHRONISCHEN SCHMERZSYNDROMEN // CONTRALATERAL LOCAL ANESTHESIA IN ACUTE AND CHRONIC PAIN SYNDROMES" ANAESTHESIST, SPRINGER VERLAG, DE, Bd. 34, Nr. 10, 1. Januar 1985 (1985-01-01), Seite 551, XP009125882 ISSN: 0003-2417
- MELLICK LARRY B ET AL: "Treatment of headaches in the ED with lower cervical intramuscular bupivacaine injections: A 1-year retrospective review of 417 patients" HEADACHE, WOODBURY, NJ, Bd. 46, Nr. 9, 1. Oktober 2006 (2006-10-01), Seiten 1441-1449, XP009125859 ISSN: 0017-8748
- SATO CHIYO ET AL: "The prolonged analgesic effect of epidural ropivacaine in a rat model of neuropathic pain" ANESTHESIA AND ANALGESIA, WILLIAMS AND WILKINS, BALTIMORE, MD, US, Bd. 106, Nr. 1, 1. Januar 2008 (2008-01-01), Seiten 313-320, XP009125860 ISSN: 0003-2999

## Beschreibung

Die vorliegende Erfindung beschäftigt sich mit der Verwendung von Lidocain oder dessen 3-OH- oder 4-OH-Derivaten, in einer Konzentration von 0,05 Gew.-% bis 0,2 Gew.-%, zur Behandlung oder Prophylaxe von chronischen Erkrankungen oder (chronischen) Kopfschmerzerkrankungen, insbesondere der Therapie von chronischen bzw. wiederkehrend auftretenden Schmerzen, vor allem Kopfschmerzen, z.B. bei Migräne mit und ohne Aura, retinaler Migräne, chronischer Migräne, Kopfschmerzen vom Spannungstyp, anderen primären (auch juvenilen) Kopfschmerz-Syndromen, oder sonstigen (chronisch) auftretenden (Kopf)schmerzerkrankungen. Die Erfindung beschreibt des weiteren pharmazeutische Zusammensetzungen.

Die Behandlung von Schmerzen im Allgemeinen ist ein in der heutigen Medizin häufig auftretendes Erfordernis, sowohl als begleitende Therapie, z.B. bei der Vornahme invasiver Eingriffe, oder als Primärtherapie. Schmerz kann dabei allgemein in akute und chronische Schmerzen unterschieden werden, wobei akute Schmerzen im üblichen als Warnsignal dienen und damit einen wertvollen Hinweis zur Diagnose der zugrundeliegenden Krankheit liefern. Akute Schmerzen haben dabei üblicherweise eine zumeist eindeutig bestimmbare Ursache, wobei eine kausale, d.h. ursachenorientierte Behandlung typischerweise zu einem Nachlassen und nach einer gewissen Zeit zum Verschwinden des Schmerzes führt. Im Unterschied dazu überdauern chronische Schmerzen diesen Zeitraum, in dem üblicherweise eine Heilung der für die Schmerzen verantwortlichen Ursache stattfindet. Primär chronische Schmerzen umfassen beispielsweise Migräne, Cluster-Kopfschmerz, Trigeminusneuralgie, postzosterische Neuralgie, Stumpf- und Phantomschmerzen, Thalamusschmerz und tumorbedingte Schmerzen. Gerade bei solchen chronischen Schmerzen aber auch bei Akutschmerzen, die nicht nach der zu erwartenden Zeit zu beseitigen sind und damit in den chronischen Zustand übergehen können, müssen Behandlungsmaßnahmen ergriffen werden, die präventiv wirken, also der Entwicklung der Schmerzkrankheit entgegenwirken können. Chronische Schmerzen haben - im Gegensatz zu akuten Schmerzen - jedoch fast nie nur eine einzige auslösende oder unterhaltende Ursache, sie sind multikausal. Eine der besonders häufig auftretenden Formen des primär chronischen Schmerzes ist die Migräne. Die Migräne ist eine neurologische Erkrankung, unter der etwa 10 % der Bevölkerung leiden. Die Migräne, die bei Frauen etwa dreimal häufiger auftritt als bei Männern, hat ein vielgestaltiges Krankheitsbild. Dieses ist bei Erwachsenen typischerweise durch einen periodisch wiederkehrenden, anfallartigen, pulsierenden und halbseitigen Kopfschmerz gekennzeichnet, der von zusätzlichen Symptomen wie Übelkeit, Erbrechen, Lichtempfindlichkeit (Photophobie) oder Geräuschempfindlichkeit (Phonophobie) begleitet werden kann. Bei manchen Patienten geht einem Migräneanfall eine Migräneaura voraus, während derer häufig optische oder sensible Wahrnehmungsstörungen auftreten, aber auch motorische Störungen auftreten können. Die Diagnose wird nach Ausschluss anderer Erkrankungen üblicherweise mit Hilfe einer Anamnese gestellt.

Die Behandlung akuter Schmerzen unterscheidet sich zu der Behandlung chronischer Schmerzen insbesondere in der Art, aber auch der Dosierung der bei der Behandlung eingesetzten Wirkstoffe. So werden bei der Schmerzausschaltung bei akuten Schmerzen, aber auch in anderen Gebieten, wie z.B. der Kardiologie, mittlerweile routinemäßig Lokalanästhetika wie z.B. Articain, Bupivacain, Lidocain, Mepivacain, Prilocain, Procain, Tetracain, etc., zumeist in recht hohen Konzentrationen von 0,1 bis 0,5 Gew.-% oder höher eingesetzt. Die hierbei eingesetzten Lokalanästhetika können allgemein in Lokalanästhetika des Amid- und Estertyps klassifiziert werden, wobei sich Lokalanästhetika vom Estertyp noch weiter in Ester der Aminobenzoesäure und Benzoesäureester unterscheiden lassen.

Lokalanästhetika wirken im Allgemeinen durch die Blockade der Erregungsleitung, wobei in Anwesenheit des Lokalanästhetikums die ankommende Erregungswelle, das Aktionspotential, nicht mehr zu depolarisieren vermag, d.h. die Depolarisationswelle ist an dieser Stelle nicht mehr in der Lage, die Na*-Permeabilität so zu erhöhen, dass der für die Fortpflanzung der Erregungswelle notwendige Grad der Depolarisation zustande kommt. Die Ursache dafür liegt typischerweise in einer Blockade des spannungsabhängigen Natriumkanals durch das Lokalanästhetikum.

Solche Lokalanästhetika werden allgemein in der Therapie von akuten Schmerzen, wie bspw. bei der Beseitigung von Schmerz- und Juckreizen, bei ophtalmologischen Operationen, zur Zahnbehandlung, oder bei gynäkologischen, urologischen oder chirurgischen Eingriffen verwendet, sind jedoch in der Behandlung von chronischen Schmerzen kaum bekannt und werden dort üblicherweise nicht eingesetzt.

So beschreibt beispielsweise die DE 24 04 620 eine Infusionslösung zur Behandlung tachykarder ventrikulärer Herzrhythmusstörungen und tachykarder Arrhythmien. Die in DE 24 04 620 gezeigte Infusionslösung zur intravenösen Injektion weist dabei einen Gehalt an Lidocain von 2000 mg/l bzw. 0,5 - 5 g/l auf.

Die Veröffentlichung DE 101 97 266 (WO 02/22128) zeigt bspw. eine Zusammensetzung umfassend Tetracain als herkömmliches örtliches Betäubungsmittel für akute Schmerzen in einer Konzentration von 0,2 bis 5 Gewichtsprozent. Die in DE 101 97 266 beschriebene Lösung dient zur topischen Verabreichung in Verfahren zur örtlichen Betäubung und akuten Schmerzstillung.

Lokalanästhetika vom Amid- und Estertyp, bspw. Lidocain und Benzocain, werden ferner in der DE 10 2004 001 093 beschrieben und in einer Konzentration von 0,5 bis 40% zur Behandlung akuten neuromuskulären Schmerzen, sowie Gelenkschmerzen nach Prellungen, Dehnungen und Zerrungen der Muskulatur eingesetzt, insbesondere zur lokalen Schmerzstillung, ausschließlich durch topische Verabreichung. Die DE 10 2004 001 093 beschreibt ebenfalls die topische Behandlung akuter rheumatischer Muskelbeschwerden und von akuten postincisionalen und post-operativen Wundschmerzen. In der Anmeldung DE 10 2004 027 685 des gleichen Anmelders werden zudem Lokalanästhetika vom Amid-und Estertyp in einer Konzentration von 0,1-30% ebenfalls zur topischen Behandlung von überempfindlichen Zähnen und Dentinschmerz offenbart.

Auch das erteilte Patent DE 601 09 164 (EP 01 948 733.9) offenbart Zusammensetzungen mit 0,01 bis 0,5 % Lokalanästhetika vom Amid- und Estertyp, ausgewählt aus Lidocain, Tetracain, Prilocain, Bupivacain, Mepivacain, Etidocain, Procain, Benzocain, Chloroprocain, Ropivacain und Dibucain, ausschließlich zur topischen Verabreichung, bei der Behandlung von Schmerzen der Schleimhaut.

In der Patentschrift DE 696 30 947 (EP 96 911 538) werden 3-OH- und 4-OH-Derivate der Verbindungen Lidocain, Mepivacain, Bupivacain, Etidovacain und Prilocain beschrieben. Die DE 696 30 947 beschreibt zusätzlich die Verwendung dieser Verbindungen in einer Konzentration von etwa 0,1 Gew.-% bis etwa 90 Gew.-% des therapeutischen Mittels zur systemischen Verabreichung mittels intravenöser Injektion.

Die EP 1 300 141 zeigt die Behandlung von verletztem rektalem submukosalem Gewebe mittels Injektion einer Injektionslösung enthaltend 0,1 bis 1 % Lidocain oder 0,5 bis 5% Procain. Die EP 1 300 141 beschreibt dabei einen bevorzugten Konzentrationsbereich von etwa 0,5 Gew.-% für Lidocain oder Procain.

In der WO 90/00390 wird weiterhin die Behandlung postoperativer akuter Schmerzzustände beschrieben, wobei hier Dosierungen von etwa 5 mg/l für Bupivacain, 50 mg/l für Lidocain und 7,5 mg/l für Ropivacain genannt werden.

So wird bspw. in der wissenschaftlichen Veröffentlichung Koppert et al. (Pain 85, 2000, pp. 217-224) die Behandlung neuropathischer Erkrankungen und Schmerzzustände, insbesondere die Behandlung von Hyperalgesie, d.h. einer übermäßigen Schmerzempfindlichkeit und Reaktion auf einen üblicherweise (akuten) schmerzhaften Reiz, sowie zur Spinalanästhesie, durch die systemische Verabreichung einer ersten Dosis von 2 mg/kg Lidocain, gefolgt von einer zweiten Dosis Lidocain von 2 mg/kg•h innerhalb von 10 min beschrieben.

Des Weiteren wird in einem Kurzbericht von Brofeldt et al. (Academic Emergency Medicine 5(12), 1998, pp. 1224-1229) die perikraniale Injektion von Lokalanästhetika untersucht, wobei sich eine effektive Behandlung von wiederkehrendem Kopfschmerz ergab, welcher mit Standardverfahren nur unzureichend behandelt werden konnte.

Ashkenazi et al. (Neurology 66(5.2), 2006, p. A223) evaluieren die Effizienz von Lidocain und Bupivacain mit oder ohne die Gabe von Kortikosteroiden für die Behandlung chronischer Kopfschmerzen: die Untersuchungen zeigen, dass die Gabe von Kortikosteroiden keinen zusätzlichen Nutzen ergibt.

Katsushi et al. (Journal of Clinical Anaesthesia 14(1), pp. 39-41) berichten über die effektive Behandlung von Neuralgien unter Verwendung von Tetracain (5 %).

Akute und chronische Schmerzsyndrome (Phantom- oder Stumpfschmerz) konnten von Gross (Anaesthesist 34(10), 1985, p. 551) mit kleinen Dosen des Lokalanästhetikums (0,1 - 1,0 ml 1 %iges Mepivacain) in ca. 80 % der Patienten nachweisbar effektiv behandelt werden.

Nicht näher spezifizierte Kopfschmerzen konnten gemäß Mellick et al. (Headache 46(9), 2006, pp. 1441-1449) durch zervikale intramuskuläre Injektion mit Bupivacain (0,5 %) erfolgreich behandelt werden.

Von einem lang anhaltenden analgetischen Effekt nach wiederholter epiduraler Gabe des Lokalanästhetikums Ropivacain (0.2 %) bei der Behandlung von neuropathischen Schmerzen im Rattenmodell berichten Chiyo et al. (Anaesthesia and Analgesia 106(1), 2008, pp. 313-320).

In der Patentschrift WO 99/03473 wird eine pharmazeutische Zusammensetzung enthaltend Ropivacain (0,01 - 53 %), eine Methode sowie ein Kit für die Behandlung zerebraler neurovaskulärer Krankheitszustände wie Migräne oder Cluster-Kopfschmerzen bzw. muskulärer Kopfschmerzen wie Spannungskopfschmerz und Kopfschmerz, welcher durch Muskelkontraktion entsteht, beschrieben. Die Behandlungsmethode umfasst insbesondere die intranasale Verabreichung.

Lokalanästhetika werden daher als Mittel zur Schmerzausschaltung vor allem bei akuten Schmerzen eingesetzt, z.B. bei medizinischen Prozeduren, wie invasiven Eingriffen, Chirurgie, in der Schmerztherapie gute Erfolge lieferten. Lokalanästhetika werden ferner auch in der Neuraltherapie, in der Orthopädie und Ophthalmologie sowie in der Zahnmedizin und insbesondere in der Anästhesie eingesetzt, jedoch auch hier zur Behandlung von akuten Schmerzen.

In diesem Zusammenhang ist es relevant, dass Lokalanästhetika ihre Wirkung typischerweise an der Zellmembran von Nervenzellen entfalten. Die in der Humanmedizin üblicherweise verwendeten Lokalanästhetika der Amid- und Estergruppe blockieren Natriumkanäle und verhindern dadurch den Einstrom von Natriumionen in die Zelle und die Bildung von Aktionspotentialen. Damit werden die Ausbildung und Weiterleitung von Empfindungen wie Temperatur, Druck oder Schmerz und die Überleitung motorischer Impulse abgeschwächt oder teilweise sogar ganz unterbrochen. Typische Lokalanästhetika liegen zudem bei physiologischem pH-Wert typischerweise in geladener und ungeladener Form vor. Der Wirkort dieser Lokalanästhetika liegt typischerweise an der Innenseite der Natriumkanäle in der Zellmembran, was eine Diffusion der Substanzen in das Zellplasma erfordert. Wichtig ist daher die Gesamtladung des *in vivo* vorliegenden Lokalanästhetikums. Nur die ungeladene Base kann durch die Zellmembran ins Innere gelangen, dissoziiert hier in die geladene Form, welche zumeist auch die aktive Form des Lokalanästhetikums darstellt, gelangt an die Bindungsstelle des Natriumkanals und entfaltet dort ihre Wirkung. Die Blockierung von Ionenkanälen und damit der elektrischen Aktivität von Nervenzellen und deren Signalübertragung durch Lokalanästhetika ist damit im Wesentlichen abhängig von den molekularen Eigenschaften des Lokalanästhetikums. Andere molekulare Eigenschaften außer der Ladung sind die Konzentration, die Art und der Zustand des Gewebes, in dem die hemmende Wirkung entfaltet werden soll und die aktuelle enzymatische Aktivität innerhalb der Ionenkanäle. Letztlich ist die Signalübertragung durch Lokalanästhetika daher zunächst vom Alter und gesundheitlichen Allgemeinzustand des zu Behandelnden abhängig.

Da die Signalübertragung bestimmter Nerven in bestimmten Geweben für die Steuerung komplexer Vorgänge innerhalb der durch die Nerven verbundenen Gewebe verantwortlich ist, darunter vorrangig Rückenmark und Gehirn, kann, wie die Erfinder nunmehr festgestellt haben, die gezielte Beeinflussung dieser Signalübertragung darüber hinaus der Besserung oder Behebung auch (chronisch) krankhafter und insbesondere (chronisch) schmerzhafter, Zustände ebenso dienen wie der Unterdrückung von Schmerzen oder aktuell unerwünschter muskulärer Aktivität bei invasiven Eingriffen. Neben der direkten Signalhemmung eines einzelnen Nervs können Lokalanästhetika dabei auch Auswirkungen auf andere Bereiche des den Nerv umgebenden Gewebes oder auf andere Gewebe aufweisen. So produzieren beispielsweise Schmerz-leitende Nerven regelmäßig entzündungsauslösende Eiweißkörper, die bei chronischer Reizung dieser Nerven freigesetzt werden und in betroffenen Geweben Entzündungskaskaden auslösen können. Solche Entzündungskaskaden werden im Allgemeinen als sogenannte neurogene Entzündungen charakterisiert. Durch die Signalhemmung eines Nerven mit Lokalanästhetika, die mit der Blockierung der Informations-Übertragung innerhalb des Nerven gleichzusetzen ist, kann gleichzeitig die Produktion von Entzündungseiweißen in der Nervenzelle und in ihrer Umgebung inhibiert oder zumindest deutlich gesenkt werden (de Klaver MJ, Buckingham MG, Rich GF: Lidocaine attenuates cytokine-induced cell injury in endothelial and vascular smooth muscle cells. Anesth Analg. 2003 Aug;97(2):465-70, table of contents; Lahav M, Levite M, Bassani L, Lang A, Fidder H, Tal R, Bar-Meir S, Mayer L, Chowers Y: Lidocaine inhibits secretion of IL-8 and IL-I beta and stimulates secretion of IL-I receptor antagonist by epithelial cells. Clin Exp Immunol. 2002 Feb; 127(2):226-33; Shiga M, Nishina K, Mikawa K, Obara H: The effects of lidocaine on nitric oxide production from an activated murine macrophage cell line. Anesth Analg. 2001 Jan;92(1):128-33). Die Verwendung von Lokalanästhetika erlaubt daher über diesen Mechanismus eine günstige Beeinflussung des Verlaufs von Entzündungen und damit auch des von ihnen induzierten Krankheitsgeschehens. Da neurogene Entzündungen nach neueren wissenschaftlichen Untersuchungen im Zentrum vieler zellpathologischer Veränderungen mit weitreichenden, häufig organübergreifenden, Konsequenzen stehen, ist dem gezielten Einsatz von Lokalanästhetika eine immer größere Bedeutung zuzuschreiben. So macht man sich diese Effekte zum Teil in der Neuraltherapie zunutze, die, wie oben beschrieben, zumeist mit Lokalanästhetika in handelsüblichen Konzentrationen arbeitet, im allgemeinen in einem Bereich von etwa 0,5 Gew.-% bis 1 Gew.-% oder mehr.

Es hat sich nunmehr aber ebenfalls erwiesen, dass der Einsatz der klassischen Neuraltherapie insbesondere bei Spätstadien chronischer Erkrankungen, die durch chronisch rezidivierende oder perpetuierende, häufig multifokale Entzündungen gekennzeichnet sind und damit auch Nerven schädigen können, vielfach nur begrenzt wirksam bzw. in manchen Fällen völlig wirkungslos ist. Die Gründe für ein solches Versagen sind äußerst komplex. Stark vereinfacht lassen sich dafür als mögliche Ursachen im Wesentlichen zellbiologische und neurobiologische Mechanismen beschreiben. Zellbiologische Mechanismen sind in chronisch entzündeten Geweben v.a. durch strukturmorphologische Veränderungen der Zellwände, wie z.B. Zellwandverdickungen und Verplumpungen filigraner Mikrostrukturen innerhalb der für Stoffaustausch und Signalübertragung verantwortlichen Zellwandkanäle, gekennzeichnet. Solche strukturmorphologischen Veränderungen der Zellwände führen in der Folge zu einer Beeinträchtigung der intrazellulären/extrazellulären sowie der transaxonalen molekularen Transportkapazität mit konsekutiver Störung der intrazellulären und interzellulären Signalübertragung. Da die Neuraltherapie hinsichtlich ihrer Wirksamkeit jedoch auf eine hinreichend intakte Signalübertragung angewiesen ist, führt die Verwendung von Lokalanästhetika aufgrund von solchen zellbiologischen Mechanismen dann zumeist nicht mehr zum gewünschten Therapieerfolg. Eine weitere Ursache für ein Versagen der Verwendung von Lokalanästhetika bei diesen Indikationen können neurobiologische Mechanismen sein. Neurobiologische Mechanismen, wie bspw. eine erfolgreiche neuraltherapeutische Intervention, z.B. die Behandlung von (Schmerz-) Triggerpunkten, sind üblicherweise abhängig von a) einer noch weitgehend intakten interneuronalen Signalübertragung im Rückenmark und ihrer Fortleitung in die supraspinalen Zielorgane, darunter Blutgefäße und Neuronenverbände des Gehirns, und b) der aktuell pathogenetischen Relevanz ausgewählter Injektionsorte. Bei chronisch rezidivierenden oder perpetuierenden multifokalen bzw. konfluierenden Entzündungen und sich daraus verhältnismäßig häufig entwickelnden, in weit entfernte Körperregionen übertragenen, Schmerzen und Entzündungen finden neuraltherapeutische Interventionen allerdings häufig ihre Zielorgane nicht mehr oder ausschließlich Ziele an der Peripherie pathogenetisch relevanter Strukturen. Beispielsweise finden sich bei einem Migräneanfall häufig hochschmerzhafte Nervenaustrittspunkte des Gesichtsnerven auf der Anfallsseite. An dieser Stelle vorgenommene neuraltherapeutische Infiltrationen bleiben jedoch in aller Regel wirkungslos.

Eine klinisch wirksame Behandlung chronischer, chronisch rezidivierender und neuropathischer Erkrankungen ist unter neuroanatomischen Gesichtspunkten nur möglich, wenn man die pathogenetisch relevanten Neuronenverbände, die aus den unterschiedlichsten peripheren Regionen zum Rückenmark ziehen und nach intraspinaler Umschaltung auf ihre Zielneurone das Rückenmark wieder verlassen, sowohl prä- als auch postspinal infiltriert. Zu diesen Neuronenverbänden sind vor allem auch die prä- und postganglionären sympathischen Neurone zu zählen, die bei der Chronifizierung von Erkrankungen neueren wissenschaftlichen Erkenntnissen zufolge von herausragender pathogenischer Bedeutung sind. Zur Erzielung eines bestmöglichen Behandlungsergebnisses ist typischerweise die Repetition der Maßnahmen anfangs in Zeitabständen von ca. 8 bis 12 Stunden, später von ca. 24 Stunden erforderlich.

Rückenmarksnahe Gewebe sind extrem dicht und plurineural benervt. Es wird daher allgemein eher angenommen, dass eine lokal verhältnismäßig hohe Konzentration von Lokalanästhetika zum gewünschten Therapieerfolg führt. Üblicherweise liegen die Konzentrationen von Lokalanästhetika in einem Bereich von mindestens 0,5 Gew.-% bis 1 Gew.-%, zumeist jedoch deutlich über diesem Bereich. Mit solchen handelsüblichen Konzentrationen lassen sich jedoch unerwünschte Wirkungen der verwendeten Lokalanästhetika, wie Sensibilitätsstörungen, Störungen der autonomen Regulation und partielle Paresen, wie sie bereits aus der Lumbal- und Peridural-Anästhesie bekannt sind, nicht vermeiden. Da eine einzelne, segmentgebundene Behandlung wegen der intersegmentalen neuronalen Verflechtungen üblicherweise keinen vollständigen Behandlungserfolg herbeiführen kann, ist man zudem auf Mehrfachinfiltrationen angewiesen. Insbesondere solche Mehrfachinfiltrationen führen jedoch bei Verwendung handelsüblicher Lokalanästhetika in solch üblichen hohen Dosierungen zu den beschriebenen zentralnervösen Nebenwirkungen der Lokalanästhetika und stehen damit unter einem besonderen Risiko. Durch solche Nachteile wird die methodische Überlegenheit der vorstehend skizzierten Behandlungen pharmakologisch in Frage gestellt.

Die Grenzen des Einsatzes bekannter Lokalanästhetika nach bisherigen Behandlungsschemata sind damit, nicht nur in der Neuraltherapie, methodisch limitiert. Zusätzlich zur methodischen Limitierung zeigen sich jedoch auch auf pharmakologischer Ebene deutliche Beschränkungen. Beispielsweise begrenzt die molekular- und konzentrationsabhängige zytotoxische Wirkung von Lokalanästhetika deren Einsatz in der Medizin. Zwar wäre für viele, insbesondere chronisch krankhafte Zustände, eine länger andauernde Signalhemmung Schmerz-leitender Nerven überaus wünschenswert. Jedoch können z.B. Lokalanästhetika bestimmter Konzentration, Art und Wirkdauer regelmäßig zu einer erheblichen Schädigung der Zellatmung sowie zum Zelltod führen (Johnson ME, Uhl CB, Spittler KH, Wang H, Gores Gj: Mitochondrial injury and caspase activation by the local anesthetic lidocaine. Anesthesiology. 2004 Nov; 101 (5): 1 184-94). In Abhängigkeit der Funktion betroffener Zellen bzw. Zellverbände kann dies, wie Beispiele von Herz und Hirn zeigen, unter Umständen auch den Tod des gesamten Organismus zur Folge haben.

Ausgehend von den im Stand der Technik gezeigten Limitierungen bei der Verabreichung von Lokalanästhetika lag daher der vorliegenden Anmeldung die Aufgabe zugrunde, neue Mittel oder Zusammensetzungen, enthaltend Lidocain oder ein 3-OH- oder 4-OH-Derivat davon zur Verfügung zu stellen, insbesondere neue Dosierungen und Formulierungen, die in deutlich verringertem Maße oder bevorzugt gar nicht mehr methodische und pharmakologische Limitierungen bei der Behandlung von chronischen Erkrankungen, insbesondere chronischen Schmerzzuständen, aufweisen. Bevorzugt sollen solche neuen Mittel oder Zusammensetzungen geeignet sein, die Signalübertragung derjenigen sympathischen Nerven zu beeinflussen, die mit den bei chronischen Schmerzzuständen dominant involvierten Schmerz-leitenden Nerven vom C-Faser-Typ nahezu baugleich sind, womit die wirksame Behandlung von solchen Erkrankungen möglich wird, die mit schwerwiegenden und häufig therapieresistenten neuro- und psychovegetativen Symptomen einhergehen, insbesondere von chronischen Schmerzzuständen wie z.B. Kopfschmerz und Migräne.

Die Erfinder der vorliegenden Erfindung haben dabei überraschenderweise festgestellt, dass die Verwendung von Lidocain und dessen Derivaten, bei einer niedrig-dosierten Verabreichung typischerweise unter den üblicherweise verwendeten Konzentrationen, zu einer drastischen Verbesserung bei der Therapie von chronischen Erkrankungen, insbesondere bei Migräne und chronischen Kopfschmerzerkrankungen führt.

Diese Aufgabe wird daher erfindungsgemäß durch die Verwendung von niedrig-dosiertem Lidocain gelöst, oder einem 3-OH- oder 4-OH-Derivat davon, in einer Konzentration von 0,05 Gew.-% bis 0,2 Gew.-%, zur Herstellung einer pharmazeutischen Zusammensetzung zur Therapie von chronischen Schmerz- oder Kopfschmerzerkrankungen durch Infiltrations-Anästhesie.

Das niedrig-dosierte Lidocain ist bevorzugt ausgewählt aus3-OH- oder 4-OH-Derivaten. Ein "Derivat" umfasst in diesem Zusammenhang alle Salze der3-OH- oder 4-OH-Derivate, insbesondere Hydrochloride von Lidocain, sowie weitere von Lidocain abgeleitete Verbindungen wie z.B. N-(2,6-Dimethylphenylcarbamoylmethyl) triethylammoniumbromid (QX-314). Besonders bevorzugt wird für die erfindungsgemäße pharmazeutische Zusammensetzung z.B. ein Salz von Lidocain, wie bspw. Lidocainhydrochlorid, oder N-(2,6-Dimethylphenylcarbamoylmethyl) triethylammoniumbromid (QX-314) eingesetzt.

In der erfindungsgemäß verwendeten pharmazeutischen Zusammensetzung wird Lidocain oder ein Derivat davon eingesetzt, z.B. ein Salz von Lidocain, wie bspw. Lidocainhydrochlorid, oder N-(2,6-Dimethylphenylcarbamoylmethyl)triethylammonium-bromid (QX-314). Die Verwendung von Lidocain und seinen 3-OH- oder 4-OH-Derivaten wird besonders durch dessen Mehrfachnutzen in der Heilungsförderung [Hollmann et al., Anesthesiology, 2004 Apr, 100(4):852-60; Hollmann et al., Anesthesiology, 2002 Dec, 97(6):1451-7], bei der besonderen Wirksamkeit bei neuropathischen Schmerzen [Hiruma et al., Acta Neurobiol Exp (Wars). 1999, 59(4):323-7; Koppert et al., Pain. 2000 Mar, 85(1-2):217-24; Mao et al., Pain. 2000 Jul, 87(1):7-17; Zhang et al., Pain. 2004 May, 109(1-2): 143-9] und deren Prävention [Smith et al., Pain. 2002 Jun, 97(3):267-73] gestützt. Diese Beobachtungen stehen ebenfalls in Einklang mit *in vitro-* und tierexperimentellen Daten zu neu entdeckten Wirkmechanismen von Lidocain [Lei et al., Neuroscience. 2004, 125(3):691-701; Shiga et al., Anesth. Analg. 2001 Jan, 92(1):128-33; Yregard et al., Burns. 2003 Jun, 29(4):335-41].

Die Konzentrationen des niedrig-dosierten Lidocains oder eines 3-OH- oder 4-OH-Derivats davon in der erfindungsgemäßen pharmazeutischen Zusammensetzung liegen typischerweise in einem Bereich von 0,05 Gew.-% bis 0,2 Gew.% oder in einem Bereich von 0,05 Gew.-% bis 0,15 Gew.-%, bevorzugt in einem Bereich von 0,05 Gew.-% bis 0,1 Gew.-%, stärker bevorzugt in einem Bereich von 0,05 Gew.-% bis 0,09 Gew.-%, in einem Bereich von 0,05 Gew.-% bis 0,08 Gew.-%, in einem Bereich von 0,05 Gew.% bis 0,07 Gew.-%, oder in einem Bereich von 0,05 Gew.-% bis 0,06 Gew.-%.

Alternativ liegt das bei der Verwendung der oben beschriebenen pharmazeutischen Zusammensetzung eingesetzte Lidocain oder ein 3-OH- oder 4-OH-Derivat davon in einer niedrig-dosierten Konzentration von 0,05 Gew.-% bis 0,1 Gew.-% vor. Ebenfalls bevorzugt kann das bei der Verwendung der oben beschriebenen pharmazeutischen Zusammensetzung eingesetzte Lidocain oder ein 3-OH- oder 4-OH-Derivat davon in einer Konzentration von 0,075 Gew.-% bis 0,125 Gew.-% (etwa 0,1 Gew.-%, ggf. ± 0,025 Gew.-%).

Die erfindungsgemäße pharmazeutische Zusammensetzung kann dabei in flüssiger oder fester Form, als Dispersion, als Aerosol, etc. vorliegen. Die oben genannten (nicht erfindungsgemäßen) niederen Konzentrationen können in einem allgemeinen Bereich von 0,001 Gew.-% bis 0,05 sind dabei insbesondere für die Behandlung von Kindern, d.h. nicht-adulten Patienten, oder von Patienten mit Esterasemangel bzw. Esterase-Mangelerscheinungen geeignet.

Die erfindungsgemäß verwendete pharmazeutische Zusammensetzung enthält gegebenenfalls auch einen pharmazeutisch akzeptablen Träger. Der hier verwendete Begriff "pharmazeutisch akzeptabler Träger" umfasst bevorzugt einen oder mehrere kompatible feste oder flüssige Füllstoffe, bzw. Verdünnungsmittel oder einkapselnde Verbindungen, welche für die Verabreichung an einen Patienten geeignet sind. Der Begriff "kompatibel", wie hier verwendet, bedeutet, dass die Bestandteile der hier verwendeten pharmazeutischen Zusammensetzung in der Lage sind, mit dem erfindungsgemäß eingesetzten Lidocain oder einem 3-OH- oder 4-OH-Derivat davon miteinander in einer solchen Art zusammengemischt zu werden, dass keine Interaktion auftritt, welche (wesentlich) die pharmazeutische Effektivität der hier verwendeten Zusammensetzung unter gewöhnlichen Verwendungsbedingungen reduzieren würde. Pharmazeutisch geeignete Träger müssen selbstverständlich eine ausreichend hohe Reinheit und eine ausreichend geringe Toxizität aufweisen, um sie für die Verabreichung an eine zu behandelnde Person geeignet zu machen.

Die Wahl eines pharmazeutisch akzeptablen Trägers, der zusammen mit dem erfindungsgemäß verwendeten Lidocain oder einem 3-OH- oder 4-OH-Derivat davon in der hier verwendeten pharmazeutischen Zusammensetzung enthalten ist, wird grundsätzlich durch die Verwendung durch Infiltrations-Anästhesie bestimmt.
Routen zur systemischen Verabreichung im Sinne der vorliegenden Erfindung schließen transdermale, parenterale, einschließlich subkutane, intramuskuläre, und topische Routen mit ein. Besonders bevorzugt wird jedoch die Injektion des erfindungsgemäß verwendeten niedrig-dosierten Lokalanästhetikums, z.B. als pharmazeutische Zusammensetzung, an dem Ort der Schmerzentstehung oder einem definierten Injektionsort durchgeführt, d.h. nicht systemisch, sondern gezielt an oder in die entsprechende Körperstelle. Die Verabreichung durch Infiltrations-Anästhesie bringt den Vorteil der direkten Verfügbarkeit des niedrig-dosierten Lidocains oder eines 3-OH- oder 4-OH-Derivats davon am Wirkungsort und einer deutlich geringeren Dosis im Vergleich zu im Stand der Technik offenbarten Verfahren oder Verabreichungsformen. Ort der Schmerzentstehung oder Injektionsorte im Sinne der vorliegenden Offenbarung umfassen z.B. Muskel, Haut- oder Bindegewebsschicht(en), Knochen- oder Knochenhaut, z.B. an oder in bestimmte Wirbelkörper oder Nervenstränge bzw. -bahnen, insbesondere im Bereich der Halswirbelsäule, der Nackenmuskulatur, des kranio-zervikalen Übergangs (Genick), der oberen, mittleren oder unteren Brustwirbelsäule, z.B. im Bereich der Thorakalsegmente/Grenzstrangganglien T1, T2, T3, T4, T5, T6, T7, T8, T9, T10, T11, oder T12 (auch genannt Th1, Th2, Th3, Th4, Th5, Th6, Th7, Th8, Th9, Th10, Th11, oder Th12), im Bereich der Lumbalsegmente L1, L2, L3, L4 oder L5, an der Schädelbasis (ventrale Äste von C1) sowie der ventralen Äste der Spinal-Nerven C2 C3, C4, C5, C6, C7, C8, im Bereich der Hirnarterien etc. Bevorzugt umfassen solche Injektionsorte alle Nervenstränge in diesen Bereichen, bevorzugt Nervenstränge im Bereich der Nackenmuskulatur, der Thorakalsegmente T1 bis T3, des kranio-zervikalen Übergangs (Genick), im Bereich des Ganglia cervicale superius und stellatum, des splenus capitis , des semispinalis capitis, des longissimus capitis, sowie an der Schädelbasis (ventrale Äste von C1) sowie der ventralen Äste der Spinal-Nerven C2 und C3, etc.

Einige Beispiele von Verbindungen, welche als pharmazeutisch akzeptable Träger oder Bestandteile davon dienen können, sind Zucker, wie beispielsweise Lactose, Glucose und Sukrose; Stärken wie beispielsweise Kornstärke oder Kartoffelstärke; Cellulose und seine Derivate, wie beispielsweise Natriumcarboxymethylcellulose, Ethylcellulose, Celluloseacetat, pulverisiertes Tragacanth, Malz, Gelatine, Talg, feste Gleitmittel, wie beispielsweise Stearinsäure, Magnesiumstearat; Kalziumsulfat; vegetabile Öle, wie beispielsweise Erdnussöl, Baumwollsamenöl, Sesamöl, Olivenöl, Kornöl und Öl aus Theobroma; Polyole, wie beispielsweise Polypropylenglycol, Glycerin, Sorbitol, Mannitol und Polyethylenglycol; Algininsäure; Emulgatoren, wie beispielsweise Tween^{®}; Benetzungsmittel, wie beispielsweise Natriumlaurylsulfat; färbende Agenzien; Arzneistoffträger; Stabilisatoren; Antioxidantien; Konservierungsmittel; pyrogen-freies Wasser; isotonische Salzlösung und phosphatgepufferte Lösungen, etc. Zur topischen Anwendung geeignete pharmazeutisch akzeptable Träger schließen solche Träger mit ein, die zur Verwendung in Lotionen, Cremes, Gels u.ä. geeignet sind.

Die erfindungsgemäß verwendete pharmazeutische Zusammensetzung kann zusätzlich zu den oben genannten Bestandteilen solche Verbindungen enthalten, die die Aufnahme der erfindungsgemäß verwendeten pharmazeutischen Zusammensetzung bzw. des enthaltenen Lidocains oder eines 3-OH- oder 4-OH-Derivats davon durch die oder in bestimmte Membrane am Wirkungsort, ggf. aber auch durch die oder in die Haut, verstärken oder z.T. erst ermöglichen, im Folgenden Penetrationsmittel/-verstärker genannt. Unter der Aufnahme durch die oder in die Haut (Penetration) soll insbesondere die Penetration der Dermis und die Diffusion/der Transport der erfindungsgemäß verwendeten pharmazeutischen Zusammensetzung oder des darin enthaltenen Lidocains oder eines 3-OH- oder 4-OH-Derivats davon in die darunter liegenden Gewebeschichten und Blutgefäße verstanden werden. Unter der Penetration in bestimmte Membrane am Wirkungsort soll ferner die Penetration der erfindungsgemäß verwendeten pharmazeutischen Zusammensetzung oder des darin enthaltenen Lidocains oder eines 3-OH- oder 4-OH-Derivats davon in die jeweiligen, am Wirkungsort vorhandenen Membrane, Zellen, Nervenstränge, Blutgefäße, etc. verstanden werden. Solche in der erfindungsgemäßen pharmazeutischen Zusammensetzung enthaltenen Verbindungen schließen ein, ohne darauf beschränkt zu sein, Benzylalkhol, 2-(2-Ethoxy)ethanol, Propylenglykol, Methylpyrrolidon, Dimethylsulfoxid (DMSO), etc. 2-(2-Ethoxy)ethanol und Propylenglykol werden bevorzugt in einer Konzentration von 0 bis 90 Gew.-% eingesetzt, stärker bevorzugt in einer Konzentration von 20 bis 60 Gew.-%, und noch stärker bevorzugt in einer Konzentration von 25 bis 45 Gew.-%. Benzylaklohol wird bevorzugt in einer Konzentration von 0 bis 90 Gew.-%, stärker bevorzugt in einer Konzentration von 5 bis 20 Gew.-% eingesetzt. Es können alternativ oder zusätzlich auch weitere Mittel verwendet werden, die die Haut in geeigneter Weise für eine bessere Aufnahme der pharmazeutischen Zusammensetzung penetrieren, z.B. Mittel, die die Dermis und/oder Epidermis mit thermischen, optischen, mechanischen oder anderen physikalischen Mitteln penetrieren und damit einen besseren Zugang in die darunter liegenden Gewebeschichten und Blutgefäße ermöglichen. Thermische Mittel schließen ein, ohne darauf beschränkt zu sein, z.B. Hitze (Wärme) erzeugt durch Heizdrähte, durch optische Mittel, z.B. dem Einsatz eines Lasers oder anderer geeigneter Lichtquellen, oder durch Ultraschall. Optische Mittel schließen ein, ohne darauf beschränkt zu sein, z.B. Laser oder andere geeignete Lichtquellen, etc. Mechanische Mittel schließen ein, ohne darauf beschränkt zu sein, z.B. die Verwendung von Kanülen, Lanzetten, Nadeln, etc. Andere physikalische Mittel schließen ein, ohne darauf beschränkt zu sein, z.B. die Verwendung von Ultraschall, etc.

Die erfindungsgemäß verwendete pharmazeutische Zusammensetzung kann weiterhin noch Wirkstoffe enthalten, die für die Behandlung oder Prophylaxe der im folgenden genannten Indikationen geeignet sind, entzündungshemmend bzw. antiseptisch wirken, etc., z.B. Cortison, Diclofenac, Ibuprofen, etc.

Die geeignete Dosierung des verabreichten niedrig-dosiertenLidocains oder eines 3-OH-oder 4-OH-Derivats davon, bspw. in der erfindungsgemäß verwendeten pharmazeutischen Zusammensetzung, und dessen Verhältnis zum pharmazeutisch geeigneten Träger kann durch Routineexperimente mit Tiermodellen innerhalb der wie oben beschriebenen Konzentrationsgrenzen genauer bestimmt werden. Solche Modelle schließen, ohne darauf begrenzt zu sein, Modelle von Kaninchen, Schaf, Maus, Ratte, Hund und nicht-humane Primatenmodelle mit ein. Die Dosierung des in der verwendeten pharmazeutischen Zusammensetzung eingesetzten Lidocains oder eines 3-OH- oder 4-OH-Derivats davon erfolgt innerhalb der erfindungsgemäß beschriebenen Konzentrationsbereiche und umfasst üblicherweise eine sichere und effektive Menge des eingesetztenLidocains oder eines 3-OH- oder 4-OH-Derivats davon. Wie hier verwendet bedeutet "sichere und effektive Menge" eine solche Menge des eingesetztenLidocains oder eines 3-OH- oder 4-OH-Derivats davon, die ausreichend ist, um signifikant eine Schmerzempfindung des zu behandelnden Patienten während einer medizinischen Anwendung zu vermindern oder zu stoppen und/oder die Auswirkungen einer neuropathischen Erkrankung zu vermindern oder zu stoppen. Eine sichere und effektive Menge innerhalb der zuvor beschriebenen Konzentrationsbereiche vermeidet schwerwiegende Nebeneffekte wie sie im Stand der Technik bei größeren Konzentrationen, insbesondere bei den beschriebenen Indikationen, beobachtet werden. Eine solche sichere und effektive Menge eines wie hier beschriebenen niedrig-dosierten Lidocains oder eines 3-OH- oder 4-OH-Derivats davon oder Wirkstoffs im Allgemeinen wird, innerhalb des Bereichs vernünftiger medizinischer Urteilskraft, im Zusammenhang mit dem besonderen zu behandelnden Zustand variieren, sowie dem Alter und dem physischen Zustand des zu behandelnden Patienten, der Schwere des Zustandes, der Dauer der Behandlung, der Natur der begleitenden Therapie, des verwendeten besonderen pharmazeutisch geeigneten Trägers und ähnlichen Faktoren innerhalb des Wissens und der Erfahrung des begleitenden Arztes.

Der erfindungsgemäße Einsatz des verabreichten niedrig-dosiertenLidocains oder eines 3-OH- oder 4-OH-Derivats davon, bspw. in der erfindungsgemäß verwendeten pharmazeutischen Zusammensetzung, erlaubt einmalige oder mehrmalige Gaben des verabreichten niedrig-dosiertenLidocains oder eines 3-OH- oder 4-OH-Derivats davon, z.B. die einmalige oder mehrmalige Verabreichung der erfindungsgemäßen pharmazeutischen Zusammensetzung bspw. pro Tag, pro Woche oder pro Monat. Die Verabreichung kann bspw. 1-5 mal, d.h. 1, 2, 3, 4, oder 5 mal, 5-10 mal, d.h. 5, 6, 7, 8, 9 oder 10 mal, 10 bis 20 mal, 20 bis 40 mal oder häufiger pro Tag, pro Woche, pro Monat oder pro Jahr stattfinden. Die Verabreichung kann kontinuierlich oder diskontinuierlich erfolgen. Eine kontinuierliche Verabreichung bedeutet vorliegend, dass die erfindungsgemäße pharmazeutische Zusammensetzung ohne Unterbrechung der Verabreichung an einen Patienten verabreicht wird. Bei einer diskontinuierlichen Verabreichung dagegen wird die Verabreichung der erfindungsgemäßen pharmazeutischen Zusammensetzung in mehreren Stufen, d.h. mit mindestens einer Unterbrechung, durchgeführt.

Die erfindungsgemäße pharmazeutische Zusammensetzung erlaubt die Verabreichung bspw. via Injektion, z.B. via transdermaler, subkutaner, intramuskulärer, bevorzugt via transdermaler, subkutraner oder intramuskulärer Injektion, bevorzugt über eine Infiltrations-Anästhesie, bspw. über eine (simultane) Mehrfachinfiltration (innerhalb einer Behandlungssitzung) ohne die zuvor beschriebenen Risiken, jedoch uneingeschränkt mit dem vorbeschriebenen Nutzen und geht damit über die Behandlungsmöglichkeiten und Behandlungsergebnisse der klassischen Neuraltherapie weit hinaus. Sie kann daher für die Heilung von Krankheiten eingesetzt werden, die bisher als unbehandelbar galten. Die erfindungsgemäße pharmazeutische Zusammensetzung kann für humane und auch für veterinärmedizinische Zwecke eingesetzt werden.

Die erfindungsgemäße pharmazeutische Zusammensetzung kann dabei in alle zuvor beschriebenen Injektionsorte verabreicht werden, insbesondere im Bereich der Halswirbelsäule, der Nackenmuskulatur, des kranio-zervikalen Übergangs (Genick), der oberen, mittleren oder unteren Brustwirbelsäule, z.B. im Bereich der Thorakalsegmente/Grenzstrangganglien T1, T2, T3, T4, T5, T6, T7, T8, T9, T10, T11, oder T12 (auch genannt Th1, Th2, Th3, Th4, Th5, Th6, Th7, Th8, Th9, Th10, Th11, oder Th12), im Bereich der Lumbalsegmente L1, L2, L3, L4 oder L5, an der Schädelbasis (ventrale Äste von C1) sowie der ventralen Äste der Spinal-Nerven C2 C3, C4, C5, C6, C7, C8, im Bereich der Hirnarterien etc. Bevorzugt umfassen solche Injektionsorte alle Nervenstränge in diesen Bereichen, bevorzugt Nervenstränge im Bereich der Nackenmuskulatur, der Thorakalsegmente T1 bis T3, des kranio-zervikalen Übergangs (Genick), im Bereich des Ganglia cervicale superius und stellatum, des splenus capitis, des semispinalis capitis, des longissimus capitis, sowie an der Schädelbasis (ventrale Äste von C1) sowie der ventralen Äste der Spinal-Nerven C2 und C3, etc.

Bevorzugte Einheitsdosisformen zur Injektion erfindungsgemäßer pharmazeutischer Zusammensetzungen schließen sterile Lösungen von Wasser, physiologische Salzlösungen oder Mischungen davon mit ein. Der pH solcher Lösungen sollte auf etwa 7,4 eingestellt werden. Geeignete Träger zur Infiltrations-Anästhesie schließen Hydrogele, Vorrichtungen zur kontrollierten oder verzögerten Freigabe, polylaktische Säure und Collagenmatrizen mit ein.

Erfindungsgemäße pharmazeutische Zusammensetzungen können daher zur Behandlung oder Prophylaxe verschiedener Indikationen, bevorzugt chronischer Schmerzerkrankungen oder (chronischer) Kopfschmerzerkrankungen verwendet werden, z.B. zur chronischen Schmerztherapie oder der Neuraltherapie, oder zur Vermeidung des Eintritts bestimmter Zustände in ein (solches) chronisches Stadium.

Ohne darauf beschränkt zu sein, werden Kopfschmerzerkrankungen oder chronische Schmerzerkrankungen, bevorzugt chronische Kopfschmerzerkrankungen, ohne darauf beschränkt zu sein, ausgewählt aus der Gruppe umfassend bspw.
primäre Kopfschmerzerkrankungen, umfassend bspw. Migräne (z.B. Migräne mit und ohne Aura, retinale Migräne, chronische Migräne), Kopfschmerzen vom Spannungstyp (z.B. Spannungskopfschmerz, Muskelkontraktionskopfschmerz, psychomyogener Kopfschmerz, stressabhängiger Kopfschmerz, gewöhnlicher Kopfschmerz, essentieller Kopfschmerz, sowie idiopathischer und psychogener Kopfschmerz), Clusterkopfschmerz (umfassend Ziliare Neuralgie, Erythromelalgie des Kopfes, Bing-Erythroprosopalgie, Hernicrania angioparalytica, Hemicrania, periodica neuralgiformis, Histaminkopfschmerz, Horton-Syndrom, Harris-Horton-Syndrom, migränöse Neuralgie nach Harris, Petrosusneuralgie nach Gerdner, Sluder-Neuralgie, Neuralgie des Ganglion sphenopalatinum, Vidianusneuralgie, ebenfalls umfassend episodische Clusterkopfschmerzen, chronische Clusterkopfschmerzen oder Clusterkopfschmerz mit gleichzeitig auftretender Trigeminusneuralgie (Cluster-Tic-Syndrom)), andere trigemino-autonome Kopfschmerzerkrankungen und andere primäre (ggf. juvenile) Kopfschmerzen (umfassend z.B. symptomatische Kopfschmerzen als sekundäre Folge einer anderen Erkrankung, paroxysmale Hemikranie, episodische paroxysmale Hemikranie, Chronische paroxysmale Hemikranie (CPH), Sunct-Syndrom (d.h. "short-lasting Unilateral Neuralgieform headache attacks with Conjunctival injection and Tearing), Suna-Syndrom (d.h. "short-lasting Unilateral Neuralgieform headache attacks with autonomic symptoms"), (wahrscheinliche) trigemino-autonome Kopfschmerzerkrankung, primärer stechender Kopfschmerz (Eispickelschmerz, Jabs-and-Jolts-Syndrom, periodische Ophthalmodynie, primärer Hustenkopfschmerz (benigner Hustenkopfschmerz, Kopfschmerz bei Valsalva-Manöver), primärer Kopfschmerz bei körperlicher Anstrengung, primärer schlafgebundener Kopfschmerz (Hypnic-Headache-Syndrom, "alarm clock" headache-Syndrom), primärer Kopfschmerz bei sexualler Aktivität, primärer Donnerschlagkopfschmerz, Hemicrania continua, oder neu aufgetretener täglicher Kopfschmerz (chronischer *de novo* Kopfschmerz, chronischer Kopfschmerz mit akutem Beginn)); oder
sekundäre Kopfschmerzerkrankungen, umfassend bspw. Kopfschmerzen, die auf Kopf-und/oder Halswirbelsäulen (Hws) -Trauma zurückzuführen sind (z.B. akuter posttraumatischer Schmerz, chronischer posttraumatischer Schmerz, akuter Schmerz nach HWS-Beschleunigungstrauma, chronischer Schmerz nach HWS-Beschleunigungstrauma, oder Kopfschmerz, zurückzuführen auf ein traumatisches intrakraniales Hämatom), Kopfschmerzen, die auf Gefäßstörungen im Bereich des Kopfes oder des Halses zurückzuführen sind (z.B. Kopfschmerz, zurückzuführen auf einen ischämischen Infarkt oder transitorische ischämische Attacken, Kopfschmerz, zurückzuführen auf eine nichttraumatische intrakraniale Blutung, Kopfschmerz, zurückzuführen auf eine nicht-ruptuierte Gefäßfehlbildung, Kopfschmerz, zurückzuführen auf eine Arteritis, A. carotis- oder A. vertebralis-Schmerz, Kopfschmerz, zurückzuführen auf eine Hirnvenenthrombose, Kopfschmerz, zurückzuführen auf andere intrakraniale Gefäßstörungen), Kopfschmerzen, die auf nicht-vaskuläre intrakraniale Störungen zurückzuführen sind (z.B. Kopfschmerz, zurückzuführen auf eine Liquordrucksteigerung, Kopfschmerz, zurückzuführen auf einen Liquorunterdruck, Kopfschmerz, zurückzuführen auf nicht-infektiöse entzündliche Erkrankungen, Kopfschmerz, zurückzuführen auf ein intrakraniales Neoplasma, Kopfschmerz, zurückzuführen auf eine intrakraniale Injektion, Kopfschmerz, zurückzuführen auf einen zerebralen Krampfanfall, Kopfschmerz, zurückzuführen auf eine Chiari-Malformation Typ I (CM1), Syndrom der vorübergehenden Kopfschmerzen und neurologischen Defizite mit Liquorlymphozytose (HaNDL), Kopfschmerz, zurückzuführen auf eine andere nicht-vaskuläre intrakraniale Störung), Kopfschmerzen, die auf eine Substanz oder deren Entzug zurückzuführen sind (z.B. Kopfschmerz, induziert durch akuten Substanzgebrauch oder akute Substanzexposition, Kopfschmerz bei Medikamentenübergebrauch, Kopfschmerz als Nebenwirkung zurückzuführen auf eine Dauermedikation (Kode zur Spezifizierung der Substanz), oder Kopfschmerz, zurückzuführen auf den Entzug einer Substanz, insbesondere Kopfschmerzen, die auf Übergebrauch, Dauereinnahme, Missbrauch oder Entzug von Suchtmitteln, Drogen oder Medikamente zurückzuführen sind), Kopfschmerzen, die auf eine Infektion (z.B. Kopfschmerz, zurückzuführen auf eine intrakraniale Infektion, Kopfschmerz, zurückzuführen auf eine systemische Infektion, Kopfschmerz, zurückzuführen auf HIV/AIDS oder chronischer postinfektiöser Kopfschmerz) oder auf eine Störung der Homöostase zurückzuführen sind (z.B. Kopfschmerz, zurückzuführen auf eine Hypoxie und/oder Hyperkapnie, Dialysekopfschmerz, Kopfschmerz, zurückzuführen auf eine arterielle Hypertonie, Kopfschmerz, zurückzuführen auf eine Hypothyreose, Kopfschmerz, zurückzuführen auf Fasten, Kopfschmerz, zurückzuführen auf eine kardiale Erkrankung oder Kopfschmerz, zurückzuführen auf eine andere Störung der Homöostase), Kopfschmerzen oder Gesichtsschmerzen, die auf Erkrankungen des Schädels sowie von Hals, Augen, Ohren, Nase, Nebenhöhlen, Zähnen, Mund oder anderen Gesichts- oder Schädelstrukturen zurückzuführen sind (z.B. Kopfschmerz, zurückzuführen auf Erkrankungen der Schädelknochen, Kopfschmerz, zurückzuführen auf Erkrankungen des Halses, Kopfschmerz, zurückzuführen auf Erkrankungen der Augen, Kopfschmerz, zurückzuführen auf Erkrankungen der Ohren, Kopfschmerz, zurückzuführen auf eine Rhinosinusitis, Kopfschmerz, zurückzuführen auf Erkrankungen der Zähne, Kiefer und benachbarter Strukturen, Kopf- und Gesichtsschmerz zurückzuführen auf Erkrankungen des Kiefergelenks, oder Kopfschmerz, zurückzuführen auf andere Erkrankungen des Schädels sowie von Hals, Augen, Ohren, Nase, Nebenhöhlen, Zähnen, Mund oder anderen Gesichts- oder Schädelstrukturen), oder Schmerzen, die auf psychiatrische Störungen zurückzuführen sind (z.B. Kopfschmerz, zurückzuführen auf eine Somatisierungsstörung, oder Kopfschmerz, zurückzuführen auf eine psychotische Störung); oder
kraniale Neuralgien, zentraler und primärer Gesichtsschmerz und andere Kopfschmerzen, umfassend bspw. kraniale Neuralgien, zentrale Ursachen von Gesichtsschmerzen, andere Kopfschmerzen sowie kraniale Neuralgien, zentraler und primärer Gesichtsschmerz (z.B. Trigeminus-Neuralgie, Glossopharyngeusneuralgie, Intermediusneuralgie, Lyryngeussuperior-Neuralgie, Nasoziliarisneuralgie, Supraorbitalisneuralgie, Neuralgien anderer orbitaler Äste, Okzipitalisneuralgie, Nacken-Zungen-Syndrom, Kopfschmerz durch äußeren Druck, kältebedingter Kopfschmerz, anhaltender Schmerz verursacht durch Kompression, Irritation oder Distorsion eines Hirnnervens oder einer der oberen zervikalen Wurzeln durch eine strukturelle Läsion, Optikusneuritis, Okulare diabetische Neuropathie, Kopfoder Gesichtsschmerz zurückzuführen auf einen Herpes zoster, Telosa-Hunt-Syndrom, Ophthalmoplegische Migräne, Kopf- oder Gesichtsschmerzen bedingt durch zentrale Ursachen, oder andere kraniale Neuralgien oder andere zentral vermittelte Gesichtsschmerzen), insbesondere Trigeminus-Neuralgie.

Erfindungsgemäße pharmazeutische Zusammensetzungen können ferner zur primären und/oder adjuvanten Therapie bei Sportverletzungen eingesetzt werden, sowie zur adjuvanten Therapie bei Strahlentherapie-induzierten Schmerzen.

Besonders vorteilhaft kann das hier beschriebene niedrig-dosierte Lidocain oder ein 3-OH-oder 4-OH-Derivat davon in den hier beschriebenen niederen Konzentrationen, z.B. in der erfindungsgemäßen pharmazeutischen Zusammensetzung, zur primären und/oder adjuvanten Therapie bei Migräne eingesetzt werden. Dies war für die Erfinder der vorliegenden Erfindung besonders überraschend.

Die Wirkung des hier beschriebenen niedrig-dosierten Lidocains oder eines 3-OH- oder 4-OH-Derivats davon in den hier beschriebenen niederen Konzentrationen bei Migräne, z.B. in der erfindungsgemäß verwendeten pharmazeutischen Zusammensetzung, beruht insbesondere auf folgenden neuroanatomischen Zusammenhängen. Migräne gilt als multifaktorielle Erkrankung, deren vielfältiger Ausprägung ein weitgehend einheitliches pathogenetisches Grundmuster zugeschrieben wird, das von intrakraniellen Veränderungen gekennzeichnet ist. Daneben werden auch neuro-anatomisch begründete Zusammenhänge mit Vorgängen an und in extrakraniellen Strukturen als mögliche pathogenetisch wirksame Mechanismen beschrieben.

Nach Erkenntnissen der Erfinder beruht die interindividuelle Vielfalt der Symptome einer Migräne auf individuell differenten, extrakraniellen Faktoren, die mehrheitlich auf die primär topisch gesteigerte Aktivität des peripheren Sympathikus zurückzuführen sind. Dabei scheinen in erster Linie funktionelle Veränderungen gelenksnaher Strukturen der Wirbelsäule Disposition und Auslösung sympathischer Überaktivität zu dominieren.

Ort und Ausprägung funktioneller Veränderungen wiederum scheinen Ort und Ausprägung intrakranieller Veränderungen und damit die Ausprägung der jeweiligen, individuell weitgehend konstanten Anfalls-typischen Symptome zu definieren. Diese Beobachtungen stimmen mit den Prinzipien der interneuronalen Zielprojektion überein. Danach werden primär afferente propriozeptive und nozizeptive somatische und viszerale Neurone auf spinaler Ebene durch spezifische Interneurone auf präganglionäre Neurone und in Grenzstrang-Ganglien auf postganglionäre sympathische Neurone zu spezifischen Zielorganen umgeschaltet. Ein solches »Zielorgan« kann auch die kurze Wegstrecke einer Arteriole sein.

Es ist weiterhin bekannt, dass sich im Migräneanfall das zerebrale Blutverteilungsmuster ändert. Die Änderungen beruhen auf primärer Vasokonstriktion sowie gegenregulatorischer Vasodilatation von Arteriolen und Kapillaren auf Wegstrecken von wenigen Millimetern Länge. Daran beteiligt sind nach den Erkenntnissen der Erfinder initial adrenerge Neurone aus den Ganglia stellatum und cervicale superius. Entsprechend der interneuronalen Zielprojektion sind einer definierten Gefäßstrecke bestimmte, primär afferente Neurone eines definierten peripheren Gewebsareals zuzuordnen. Dabei kann jedes periphere Gewebe zielprojektiv wirksam sein: Gelenkskapseln, Ligamente, Muskeln, Sehnen oder auch Gewebe innerer Organe. So konnte nach noxischer Stimulation eines Ischias-Nervs ein signifikanter Rückgang des regionalen zerebralen Blutflusses [rCBF] im Bereich von Thalamus und Hypothalamus ipsilateral beobachtet werden, kontralateral war ein moderater Rückgang des rCBF zu verzeichnen.

Die Veränderung des regionalen Blutverteilungsmusters im Migräneanfall geht demnach wohl nicht auf eine physiologische Aktivität der zentral-autonomen Regulation zurück im Sinne einer Anpassung an variable zerebrale Anforderungen, sondern erscheint als Ergebnis einer exogenen Störung mit der Folge dysregulatorischer Adaptation mit Auslösung neuroimmunologischer, neurohistochemischer und neuroneuronaler Reaktionskaskaden und Überlastung des endogenen antinozizeptiven Potentials mit der Folge von Migräneschmerzen und Migräne- und Aura-spezifischen sensomotorischen, neurovegetativen, psycho-vegetativen und koordinativen Funktionsstörungen.

Die spinale interneuronale Informationsverarbeitung beruht auf der neuro-anatomischen Konvergenz multimodaler sensorischer, primär afferenter Neurone. Die Erregung präganglionärer sympathischer Neurone ist abhängig von Anzahl und Aktivität afferenter Neurone sowie deren interneuronaler Verarbeitung. Für eine klinisch wirksame symptomauslösende bzw. symptom-modulierende intrazerebrale Vasokonstriktion kommen vor allem solche Gewebe in Betracht, die über eine hohe Dichte afferenter Neurone mit einer signifikanten Rezeptorendichte verfügen und in denen nennenswerte Zahlen präganglionärer sympathischer Neurone gefunden wurden. Die obere Brustwirbelsäule nimmt hierin eine Sonderstellung ein.

Die Erfinder haben v.a. beoachtet, dass funktionelle Veränderungen einzelner Wirbel sowie von Wirbelketten der oberen Brustwirbelsäule Aura-Symptome auslösen und Migräne-Anfälle induzieren können. Aura-Symptome wie Seh- und Sprachstörungen ebenso wie Sensibilitäts-Störungen, Hemiparesen und Hemiplegien werden in der Regel in die ersten Anfallsphasen der Migräne übernommen. Im weiteren Verlauf kann sich das primäre Anfalls-Muster jedoch auch ändern. Dies kann auf eine spontane Änderung der Körperhaltung zurückzuführen sein, vorzugsweise im Bereich der kraniozervikalen und zerviko-thorakalen Übergänge - zum Beispiel während eines Erbrechens - mit der Folge einer zumindest vorüber gehenden Beeinflussung der präexistenten, symptominduzierenden Veränderungen der Wirbelketten. Damit werden Topografie und Impulsmuster aktivierter afferenter Neurone, deren spinale Umschaltung, die Zielprojektion sympathischer Neurone und der intrazerebrale Blutfluss verändert und damit das aktuelle Anfallsmuster moduliert.

Relevant im vorliegenden Kontext ist dabei auch die Verteilung präganglionärer sympathischer Neurone. Präganglionäre sympathische Neurone projizieren zu den Ganglia cervicale superius und stellatum. Deren postganglionäre Neurone projizieren ziel-spezifisch zu den Hirnarterien. Präganglionäre sympathische Neurone werden durch segmental angeordnete Interneuronenketten aktiviert. Diese Interneurone sammeln segmental einlaufende Impulse aus multimodalen, primär afferenten Neuronen, die aus verschiedenen Segment-Höhen zu den Thorakal-Segmenten 1 bis 10 projizieren. Die Durchblutung der Hirnarterien ist aus diesen Thorakal-Segmenten beeinflussbar. Die größte Dichte präganglionärer sympathischer Neurone wurde übereinstimmend in den Segmenten T1 bis T3 gefunden. Die Segmente T1 bis T3 der oberen Brustwirbelsäule nehmen in dieser Hinsicht eine Sonderstellung ein und werden erfindungsgemäß für Injektionen der erfindungsgemäßen pharmazeutischen Zusammensetzung bevorzugt. Nach unseren klinischen Daten ist die obere Brustwirbelsäule wahrscheinlich das wichtigste Induktions-Zentrum für die Migräne-Aura und kann darüber hinaus zur Entstehung und Persistenz von Migräne-Anfällen maßgeblich beitragen.

Von großer Relevanz ist ebenfalls die Entstehung von Ischämie-Zonen in verschiedenen Regionen des Gehirns. Nach Erkenntnissen der Erfinder können neurogene Entzündungen in rezeptiven Feldern der oberen und mittleren Brustwirbelsäule proprio- und nozizeptive Neurone in Spinal-Ganglien erregen. Die Erregung wird intraspinal auf prä-ganglionäre sympathische Neurone und in den Ggl. stellatum und cervicale superius selektiv auf vasokonstriktive postganglionäre Neurone umgeschaltet. Die dadurch entstehenden Ischämie-Zonen verändern den zerebralen Blutfluss selektiv, woraus individuell konstante zerebrale Funktions-Störungen resultieren, die zur Ausbildung der typischen, individuell konstanten Migräne-(Begleit-) und Aura-Symptomatik führen können.

Bei Migräneanfällen sind weiterhin spezielle neuro-anatomische Zusammenhänge im Bereich des kranio-zervikalen Übergangs und ihre therapeutische Konsequenz in Betracht zu ziehen. Migräne-Anfälle beginnen häufig mit Schmerzen in Nacken und Hinterkopf. Soweit dieser Schmerzbeginn unter Berücksichtigung des weiteren klinischen Verlaufs dem Krankheitsbild der Migräne zugeordnet werden kann, stellt sich die Frage nach dessen Herkunft. Biomechanisch und neuro-anatomisch kommen für die Initialschmerzen in Nacken und Hinterkopf extrakranielle Mechanismen im Bereich der oberen Halswirbelsäule in Betracht - vorrangig der kranio-zervikale Übergang (Genick) sowie das Segment C3 - deren vielfältige neuro-anatomische Verbindungen über die eigentliche Schmerz-Genese hinaus auch an vestibulären Dysfunktionen beteiligt sein können, die im Rahmen von Migräne-Anfällen häufig beklagt werden.

Der kranio-zervikale Übergang (Genick) weist eine Reihe von Besonderheiten auf, die sich aus seiner embryonalen Entwicklung und biologischen Funktion ableiten lassen und sich in seiner komplexen Neuroanatomie widerspiegeln. Im Vordergrund stehen dabei die neuroanatomischen Beziehungen zu Kopf und Gehirn, insbesondere die trigemino-zervikalen Verbindungen sowie die an der Initialisierung funktions-pathologischer Veränderungen maßgeblich beteiligten Proprio- und Nozizeptoren, die in dieser Region in außerordentlich hoher Dichte beobachtet wurden. Eine weitere Besonderheit stellt die unmittelbare Nachbarschaft des kraniozervikalen Übergangs zu den Ganglia cervicale superius dar, zu denen die ihn innervierenden Nervi spinales 1 bis 3 unmittelbare Beziehungen unterhalten.

Die neuro-anatomischen Beziehungen zwischen den Strukturen des kraniozervikalen Übergangs und den Ganglia cervicale superius legen den Verdacht nahe, dass funktionspathologische Veränderungen an Strukturen des kraniozervikalen Übergangs - z.B. die akute Fehlrotation des ersten Halswirbels - zur Aktivierung postganglionärer sympathischer Neurone in Zielgebieten definierter intrakranieller Gefäße führen könnten, die prinzipiell unter dem Einfluss dieser Ganglia stehen.

Im Migräneanfall ist regelmäßig eine außerordentlich hohe Druckdolenz an den Querfortsätzen des ersten Halswirbels festzustellen, dominant auf der Anfallsseite. Der Wirbel ist dabei in der Regel funktionell unbeweglich und fehlrotiert. Die Fehlrotation ist auf Röntgenbildern, im CT und im MRT objektiv zu verifizieren. Die Dornfortsätze der 2. und 3. Halswirbel sind im Anfall ebenso auffallend druckdolent wie die Nackenmuskulatur der Region. Diesen klinischen Befunden liegen neurogene Entzündungen des periartikulären Gewebes und der Muskulatur zugrunde. Umfang und Ausdehnung dieser Entzündungen scheinen nicht nur Umfang und Ausdehnung lokaler Schmerzen in Nacken und Hinterkopf, sondern auch Art und Umfang fazialer Schmerzprojektionen sowie Intensität von Übelkeit und Schwindel zu definieren. Diese Beobachtungen sind weitgehend deckungsgleich mit den topografisch-anatomischen und neuro-anatomischen Verhältnissen dieser Region.

Angesichts der vielfältigen pathogenetischen und neuro-anatomischen Zusammenhänge war es besonders überraschend, dass das hier beschriebene niedrig-dosierteLidocain oder ein 3-OH- oder 4-OH-Derivat davon in den hier beschriebenen niederen Konzentrationen, z.B. in der erfindungsgemäßen pharmazeutischen Zusammensetzung, effektiv zur primären und/oder adjuvanten Therapie bei Migräne eingesetzt werden können und zu einer spontanen und lang andauernden Verbesserung der chronischen Schmerzen führen, in den meisten Fällen sogar zu einer Beschwerdefreiheit ohne weitere Rückfälle. Dies war besonders überraschend für die Erfinder der vorliegenden Erfindung, insbesondere angesichts der im Stand der Technik bekannten Komplikationen.

Die Verabreichung des hier beschriebenen niedrig-dosierten Lidocains oder eines 3-OH-oder 4-OH-Derivats davon in den hier beschriebenen niederen Konzentrationen, z.B. in der erfindungsgemäßen pharmazeutischen Zusammensetzung, kann, insbesondere in der Migräne-Therapie, durch Infiltrations-Anästhesie stattfinden, bspw. durch gezielte, simultane Infiltrations-Anästhesien an der Schädelbasis (ventrale Äste von C1) sowie der ventralen Äste der Spinal-Nerven C2 und C3 unter Anwendung einer pharmakologisch und technisch ziel- und zweckoptimierten Methode. Unter Einsatz der erfindungsgemäßen pharmazeutischen Zusammensetzungen lassen sich die oben beschriebenen Migräne-Schmerzen sowie die Symptome einer vestibulären Dysfunktion - Übelkeit und Schwindel - unabhängig von der vorbestehenden Anfallsdauer einer Migräne spontan und definitiv abortieren. Die Konzentration des dafür verwendeten Lidocains oder eines 3-OH- oder 4-OH-Derivats davon in der erfindungsgemäßen pharmazeutischen Zusammensetzung ist bevorzugt so eingestellt, dass keine motorischen oder Sensibilitäts-Störungen auftreten. Im Experiment an gesunden Probanden wurde z.B. eine wirksame Blockade spinaler Nozizeptoren innerhalb von 0,8 bis 1,2 Sekunden festgestellt ohne Beeinträchtigung der Sensibilität. Daher erfolgt auch die Anfallsdurchbrechung ohne Beeinträchtigung der Sensibilität in irgendeiner Körperregion.

Das unter Einsatz des hier beschriebenen niedrig-dosierten Lidocains oder eines 3-OH- oder 4-OH-Derivats davon in den hier beschriebenen niederen Konzentrationen, z.B. in der erfindungsgemäßen pharmazeutischen Zusammensetzung, erzielbaren Ergebnisse unterscheiden sich beträchtlich von denen der klassischen Migränetherapie bzw. Neuraltherapie, bei der durch Triggerpunkt-Infiltrationen im Bereich von Muskulatur und trigeminalen Nervenaustrittspunkten in der Regel nur Schmerzreduktion bei weiter bestehender Begleitsymptomatik erzielt wird.

Ohne darauf beschränkt zu sein, kann das hier beschriebene niedrig-dosierte Lidocain oder ein 3-OH- oder 4-OH-Derivat davon in den hier beschriebenen niederen Konzentrationen, z.B. in der erfindungsgemäßen pharmazeutischen Zusammensetzung, zur Behandlung weiterer chronischer Erkrankungen eingesetzt werden, bevorzugt chronischer Schmerzerkrankungen ausgewählt aus Schmerzerkrankungen, umfassend, ohne darauf beschränkt zu sein, Zoster- und postzosterische Neuralgien, chronische neuropathische Schmerzen (periphere Nervenläsionen, Wurzelläsionen) (chronische) Phantom-Schmerzen, chronische posttraumatische Schmerzen, chronische postoperative Schmerzen, chronisch auftretende Menorrhagien und Ischialgien, (chronische) LWS-Syndrome (radikulär und pseudoradikulär), chronische Schmerzen nach Bandscheibenvorfall, chronische Schmerzen bei Wirbelfrakturen, chronische Schmerzerkrankungen aufgrund von degenerativen Prozessen der Wirbelsäule, der Gelenke sowie des skeletto-muskulären Apparats, chronische Schmerzen aufgrund von (therapieresistenten) Sportverletzungen oder Tendopathien, komplexe regionale chronisch auftretende Schmerzsyndrome (CRPSI, CRPSII), chronische Schmerzen bei arbeitsbedingter Fehlhaltung (Kassiererschmerz, Computer-Maus etc.), chronische Schmerzen aufgrund von Intensionstendopathien, chronisch gewordene Tumor-/Krebsschmerzen, chronisch gewordene Schmerzen bei Tumormetastasen der Wirbelsäule, andere therapieresistente chronisch gewordene Tumorschmerzen, (z.B. Pancreaskarzinom, Ovarialkarzinom), Angina pectoris ohne Coronarsklerose, andere chronische cardiale Schmerzen ohne organische Ursache, chronische Schmerzen aufgrund von Schulter-Arm-Syndrom (radikulär und pseudoradikulär), Nackenschmerzen, akuter Herpes zoster, postzosterische Neuralgie, Intercostalneuralgie, chronische viszerale Schmerzen, chronische Schmerzformen neuroimmuner Genese, chronische urogenitale Schmerzen, chronische Schmerzen aufgrund von Arthritis, Arthrose, arterieller Verschlusskrankheit, Morbus Raynaud, etc., oder chronische Schmerzen aufgrund von sogenannten Non-pain Syndromes, also vorliegend nicht in Bezug auf Schmerz auftretende Erkrankungen, umfassend, ohne darauf beschränkt zu sein, Neurodermitis, (verzögerte) Wundheilung, Tinnitus, Hörsturz, Schwindel, Sweating etc. Insbesondere können erfindungsgemäße Zusammensetzungen z.B. zur Therapie chronischer Schmerzen, zur effektiven Schmerzausschaltung bei medizinischen Prozeduren oder in der klassischen Neuraltherapie vor allem bei Spätstadien chronischer Erkrankungen eingesetzt werden, die durch chronisch rezidivierende oder perpetuierende, häufig multifokale Entzündungen gekennzeichnet sind und damit auch Nerven schädigen können.

Das hier beschriebene niedrig-dosierte Lidocain oder ein 3-OH- oder 4-OH-Derivat davon in den hier beschriebenen niederen Konzentrationen, z.B. die erfindungsgemäße pharmazeutische Zusammensetzung, können weiterhin prophylaktisch eingesetzt werden. Z.B. kann das hier beschriebene niedrig-dosierte Lidocain oder ein 3-OH- oder 4-OH-Derivat davon in den hier beschriebenen niederen Konzentrationen dafür genutzt werden, bestimmte der oben genannten chronischen Erkrankungen ganz zu vermeiden und den Eintritt bestimmter Zustände in ein (solches) chronisches Stadium zu verhindern. Im Stand der Technik konnten solche Behandlungen nur unter den eingangs beschriebenen Nachteilen durchgeführt werden [siehe z.B. Niiyama S, Tanaka E, Tsuji S, Murai Y, Satani M, Sakamoto H, Takahashi K, Kuroiwa M, Yamada A, Noguchi M, Higashi H: Neuroprotective mechanisms of lidocaine against in vitro ischemic insult of the rat hippocampal CA1 pyramidal neurons. Neurosci Res. 2005 Nov;53(3):271-8. Epub 2005 Aug 15; Smith LJ, Shih A, Miletic G, Miletic V: Continual systemic infusion of lidocaine provides analgesia in an animal model of neuropathic pain. Pain. 2002 Jun; 97(3):267-73].

Mit Hilfe der vorliegenden Erfindung wird auch die Verwendung zur Therapie und/oder Prophylaxe der hier beschriebenen Indikationen bereitgestellt, im Folgenden "Verwendung zur (modifizierten) Infiltrations-Anästhesie" genannt.

Bevorzugt wird dabei gemäß einem ersten Schritt a) ein hier beschriebenes niedrig-dosiertes Lokalanästhetikum in den hier beschriebenen niederen Konzentrationen bzw. Dosierungen, z.B. als erfindungsgemäße pharmazeutische Zusammensetzung, wie hier beschrieben, bereitgestellt.

In einem zweiten Schritt b) wird das hier beschriebene niedrig-dosierte Lokalanästhetikum in den hier beschriebenen niederen Konzentrationen, z.B. als erfindungsgemäße pharmazeutische Zusammensetzung, an einen Patienten verabreicht, der z.B. unter einer der oben genannten Symptome leidet. Die Verabreichung der hier beschriebenen niedrig-dosierten Lokalanästhetika in den hier beschriebenen niederen Konzentrationen, z.B. als die erfindungsgemäße pharmazeutische Zusammensetzung, kann grundsätzlich transdermal, parenteral, einschließlich subkutan, intramuskulär, intraarterielloder intravenös oder topisch erfolgen.

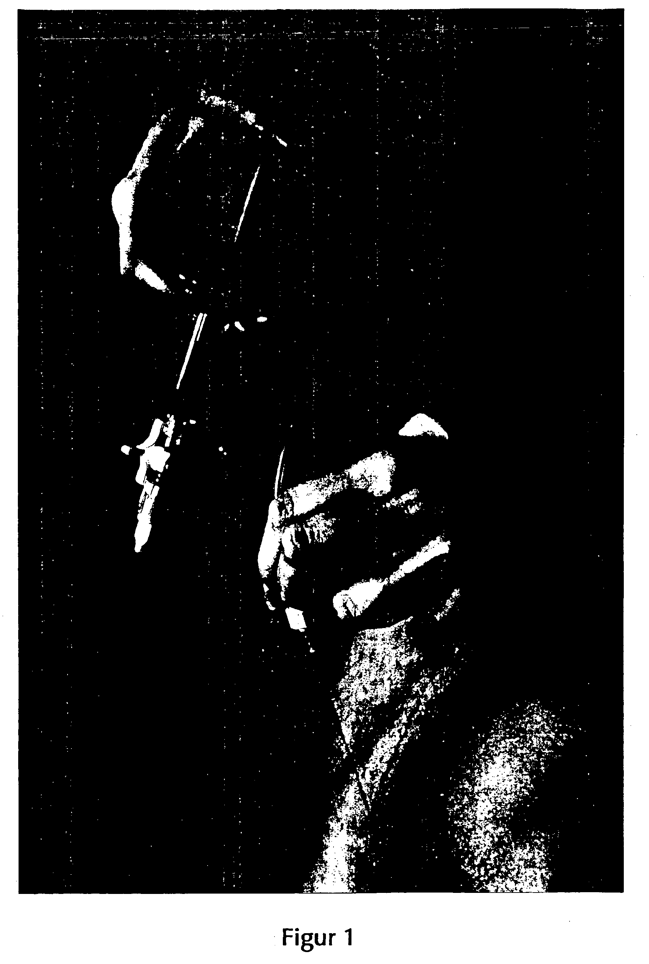

Bevorzugt wird die erfindungsgemäß verwendete pharmazeutische Zusammensetzung jedoch über eine Injektion, stärker bevorzugt über eine Injektion an den Ort der Schmerzentstehung, verabreicht. Beispielsweise kann bei Verwendung von bestimmten Injektionstechniken die erfindungsgemäß verwendete pharmazeutische Zusammensetzung durch langsamen Nadelvorschub bei gleichzeitigem, vor der Nadel ausgebrachtem schichtweisen Volumeneintrag besonders effektiv eingebracht und verabreicht werden. Die Verwendung zur (modifizierten) Infiltrations-Anästhesie zur Therapie und/oder Prophylaxe der hier genannten Indikationen umfasst die Verabreichung der hier beschriebenen niedrig-dosierten Lokalanästhetika in den hier beschriebenen niederen Konzentrationen, z.B. der erfindungsgemäßen pharmazeutischen Zusammensetzung, durch langsamen Nadelvorschub bei gleichzeitigem, vor der Nadel ausgebrachtem schichtweisen Volumeneintrag in z.B. Gewebe, bzw. einen erfindungsgemäß bevorzugten Injektionsort, eingebracht wird. Dabei werden bevorzugt alle Verfahrensschritte und Varianten eingesetzt, wie zuvor beschrieben, bspw. die zuvor beschriebenen Injektionsorte, die zuvor beschriebenen Konzentrationen, ggf. die genannten Volumina, Verabreichungszeiten und/oder Tiefe der Verabreichung. Solche erfindungsgemäß bevorzugten Injektionsorte umfassen alle zuvor beschriebenen Injektionsorte, insbesondere im Bereich der Halswirbelsäule, der Nackenmuskulatur, des kranio-zervikalen Übergangs (Genick), der oberen, mittleren oder unteren Brustwirbelsäule, z.B. im Bereich der Thorakalsegmente/Grenzstrangganglien T1, T2, T3, T4, T5, T6, T7, T8, T9, T10, T11, oder T12 (auch genannt Th1, Th2, Th3, Th4, Th5, Th6, Th7, Th8, Th9, Th10, Th11, oder Th12), im Bereich der Lumbalsegmente L1, L2, L3, L4 oder L5, an der Schädelbasis (ventrale Äste von C1) sowie der ventralen Äste der Spinal-Nerven C2 C3, C4, C5, C6, C7, C8, im Bereich der Hirnarterien etc. Bevorzugt umfassen solche Injektionsorte alle Nervenstränge in diesen Bereichen, bevorzugt Nervenstränge im Bereich der Nackenmuskulatur, der Thorakalsegmente T1 bis T3, des kranio-zervikalen Übergangs (Genick), im Bereich des Ganglia cervicale superius und stellatum, des splenus capitis , des semispinalis capitis , des longissimus capitis, sowie an der Schädelbasis (ventrale Äste von C1) sowie der ventralen Äste der Spinal-Nerven C2 und C3, etc. Die pharmazeutische Zusammensetzung ist dabei bevorzugt wie oben definiert. Der Nadelvorschub und die Injektionsgeschwindigkeit richten sich nach der Gewebekonsistenz und Patientenangaben über Druck- und/oder Schmerz-Empfinden in einzelnen Gewebsschichten während des Nadelvorschubs, bevorzugt unter Benutzung einer 10-Punkte Analog-Bewertung. Diese 10-Punkte Analog-Bewertung basiert insbesondere auf den Angaben des Patienten über Druck und/oder Schmerz zum Zeitpunkt der Injektion am Injektionsort und kann durch einen Fachmann anhand der durch den Patienten gegebenen Daten/Einschätzungen erstellt werden. Die Verabreichung erfolgt bevorzugt mit einer Nadel, bevorzugt einer Einmal-Injektionsnadel/- Kanüle, stärker bevorzugt mit einer Einmal-Injektionsnadel/-Kanüle mit einer Nadellänge in einem Bereich von 10 bis 200 mm, bevorzugt in einem Bereich von 20 bis 100 mm. Wegen der kurzfristigen Blockierung gewebsständiger Nozizeptoren, der patientenseitigen Kommentierungsmöglichkeit und der prinzipiell "stumpfen Präparation" der Gewebe durch die Injektionsflüssigkeit und nicht primär durch die Nadel ist mit dieser Technik eine gewebeschonende und zugleich komfortable Anästhesie möglich. Sie schafft vorteilhaft ebenfalls Spielraum für die gegebenenfalls erforderliche Repetition am selben Ort. Die Patientenangaben über Druck und/oder Schmerz können zur Beurteilung z.B. von Intensität und Ausdehnung neurogener Entzündungen in unterschiedlichen Gewebetiefen herangezogen werden. Dies erlaubt zugleich eine Beurteilung noch wirksamer Pathomechanismen und deren ggf. biomechanische Ursachen sowie die klinische Beurteilung des Heilungsverlaufs (vergleiche Figuren 1 bis 3).

Bei der Verabreichung der hier beschriebenen niedrig-dosierten Lokalanästhetika in den hier beschriebenen niederen Konzentrationen, z.B. als erfindungsgemäße pharmazeutische Zusammensetzung, mit Hilfe des hier beschrieben Verfahrens zur (modifizierten) Infiltrations-Anästhesie können Volumina, ohne darauf beschränkt zu sein, bspw. in einem Bereich von bspw. 1 bis 50 ml, bevorzugt in einem Bereich von 5 bis 30, und noch stärker bevorzugt in einem Bereich von 10 bis 30 ml verabreicht werden, wobei bevorzugt die oben genannten Konzentrationen zugrunde gelegt werden. Ferner können die Verabreichungen in der oben beschriebenen erfindungsgemäßen Verwendung zur (modifizierten) Infiltrations-Anästhesie, ohne darauf beschränkt zu sein, bspw. in einem Zeitraum von etwa 10 Sekunden bis etwa 10 Minuten, bevorzugt in einem Zeitraum von etwa 20 Sekunden bis etwa 5 Minuten und noch stärker bevorzugt in einem Bereich von etwa 30 Sekunden bis etwa 2 Minuten erfolgen, wobei auch hier bevorzugt die oben genannten Konzentrationen und/oder ggf. die oben genannten Volumina zugrunde gelegt werden. Schließlich können die Verabreichungen in der oben beschriebenen erfindungsgemäßen Verwendung zur (modifizierten) Infiltrations-Anästhesie, ohne darauf beschränkt zu sein, bevorzugt in die oben genannten Injektionsorte, bspw. in einer Tiefe von 0-15 cm, oder bevorzugt in einer Tiefe von 0,5 bis 10 cm oder noch stärker bevorzugt in einer Tiefe von 1 bi 7,5 cm erfolgen.

Ein weiterer Gegenstand der vorliegenden Erfindung betrifft eine kosmetische Zusammensetzung, insbesondere die Verwendung dieser kosmetischen Zusammensetzung, z.B. zur Beseitigung von kosmetischen Veränderungen, die durch die o.g. chronischen Erkrankungen oder (chronischen) Kopfschmerzerkrankungen hervorgerufen wurden oder werden, enthaltend ein wie oben beschriebenesLidocain oder ein 3-OH- oder 4-OH-Derivat davon, ausgewählt aus einem wie oben beschriebenen Lokalanästhetikum in einer wie oben beschriebene Konzentration, sowie einen kosmetisch akzeptablen Träger und optional weitere Hilfs- und Zusatzstoffe. Die erfindungsgemäße kosmetische Zusammensetzung kann in flüssiger oder fester Form, als Dispersion, als Aerosol, etc. vorliegen.

Eine "kosmetische Zusammensetzung" im Sinne der vorliegenden Erfindung ist (im Gegensatz zu einer wie oben beschriebenen erfindungsgemäß verwendeten pharmazeutischen Zusammensetzung) nicht auf die therapeutische Behandlung einer wie hier beschriebenen Erkrankung gerichtet, sondern stellt eine nicht-therapeutische Zusammensetzung bereit, die zwar ggf. einer hier beschriebenen Erkrankung vorbeugen kann, jedoch im wesentlichen, bevorzugt ausschließlich, kosmetischen Zwecken dient. Eine kosmetische Zusammensetzung, wie hier definiert, enthält ein wie zuvor beschriebenes Lokalanästhetikum oder Derivat davon und einen kosmetisch akzeptablen Träger. Ein kosmetisch akzeptabler Träger kann ein wie oben beschriebener pharmazeutisch geeigneter Träger sein. Zusätzlich kann eine erfindungsgemäße kosmetische Zusammensetzung zusätzliche, zur kosmetischen Anwendung besonders geeignete Trägerstoffe umfassen. Besonders bevorzugte Routen zur kosmetischen Anwendung schließen transdermale und/oder topische Routen mit ein. Zur topischen Anwendung allgemein und insbesondere zur kosmetischen Anwendung geeignete kosmetisch akzeptable Träger schließen solche Träger mit ein, die zur Verwendung in Lotionen, Cremes, Gels u.ä. geeignet sind. Eine kosmetische Zusammensetzung gemäß der vorliegenden Erfindung kann daher als Träger Tenside, Ölkörper, Emulgatoren, Überfettungsmittel, Perlglanzwachse, Konsistenzgeber, Verdickungsmittel, Konservierungsmittel, Parfümöle, Fette, Polymere, Stabilisatoren, biogene Wirkstoffe, übliche wasserlösliche Zusatzmittel und/oder Farbstoffe, Antischuppenmittel, Filmbildner, Quellmittel, UV-Lichtschutzfaktoren, das Fließverhalten verbessernde Stoffe, Konservierungsmittel, etc. enthalten. Zusätzlich können in erfindungsgemäße kosmetische Zusammensetzungen auch weitere Wirkstoffe eingearbeitet werden, die einen (kosmetischen) Effekt auf die Haut ausüben, z.B. natürlich vorkommende Wirkstoffe wie z.B. Kamille, Ringelblumenextrakt, Arnika Extrakt, Birkenextrakt, Brennesselextrakt, Calendulaextrakt, Grünteeextrakt, Hamamelis-Extrakt, Kamille, Meristem-Extrakt, Walnussextrakt, etc., oder Glucokorticoide wie z.B. Cortison, Hydrocortison, oder Keratolytica wie z.B. Salicylsäure, Harnstoff, etc. Die Herstellung erfindungsgemäßer kosmetischer Zusammensetzungen kann durch übliche Kalt- oder Heißprozesse erfolgen; vorzugsweise arbeitet man nach der Phaseninversionstemperatur-Methode.

Die zugrundeliegende Aufgabe wird gemäß einer anderen Ausführungsform durch Verwendung von zuvor beschriebenen Lokalanästhetika in den erfindungsgemäß beschriebenen Konzentrationen, ggf. in den genannten Volumina, ggf. den genannten Verabreichungszeiten und/oder ggf. der genannten Tiefe der Verabreichung, gelöst, bevorzugt zur Herstellung einer der zuvor beschriebenen pharmazeutischen oder kosmetischen Zusammensetzungen zur Behandlung einer der hier beschriebenen Indikationen.

Bevorzugt stellt die vorliegende Erfindung daher die Verwendung eines wie zuvor beschriebenen Lokalanästhetikums bereit, oder einem Derivat davon, in einer wie oben beschriebenen Konzentration, ggf. in den genannten Volumina, ggf. den genannten Verabreichungszeiten und/oder ggf. der genannten Tiefe der Verabreichung, zur Herstellung einer pharmazeutischen Zusammensetzung, bevorzugt wie oben beschrieben, zur Behandlung einer der oben beschriebenen Indikationen.

Ebenfalls bevorzugt stellt die vorliegende Erfindung die Verwendung eines Lokalanästhetikums bereit, ausgewählt aus den oben beschriebenen Lokalanästhetika in den oben beschriebenen Konzentrationen, ggf. in den genannten Volumina, ggf. den genannten Verabreichungszeiten und/oder ggf. der genannten Tiefe der Verabreichung, zur Herstellung einer kosmetischen Zusammensetzung, bevorzugt wie oben beschrieben.

Abschließend stellt die vorliegende Erfindung Kits, bzw. die Verwendung solcher Kits für die oben beschriebenen Anwendungen zur Verfügung, enthaltend eine erfindungsgemäß verwendete pharmazeutische oder kosmetische Zusammensetzung wie oben beschrieben, sowie gegebenenfalls einen Beipackzettel mit Instruktionen und Erklärungen zur erfindungsgemäß verwendeten pharmazeutischen oder kosmetischen Zusammensetzung.

Bevorzugt enthalten Kits mit einer erfindungsgemäß verwendeten pharmazeutischen Zusammensetzung zur Durchführung der gewünschten Behandlung, z.B. zur Verabreichung der erfindungsgemäßen pharmazeutischen Zusammensetzungen, zusätzlich geeignete Vorrichtungen. Solche zusätzlichen geeigneten Vorrichtungen können ausgewählt werden aus einer Gruppe bestehend aus:
- einer Spezial-Spritzampulle (handelsüblich), die eine wie zuvor beschriebene pharmazeutische Zusammensetzung, ggf. als Konzentrat, enthält, noch stärker bevorzugt mit Lidocain als Lokalanästhetikum;
- einer Trägerlösung, bevorzugt einer Infusionslösung wie bspw. einer Ringer-Lösung, z.B. in einer Größenordnung von 500 ml, 1000 ml, etc.;
- einer Entnahme- und Zuspritzkanüle, bevorzugt mit Griffplatte (handelsüblich);
- einer Haltevorrichtung (mit Befestigungsvorrichtung), z.B. eine Halteklaue (mit Befestigungsvorrichtung) (z.B. aus Kunststoff);
- einer oder mehreren Injektionskanülen (handelsüblich), bevorzugt 40 - 60 mm oder 70 - 100 mm;
- einem druckstabilen Überleitungsschlauch (handelsüblich); und
- einem Dreiwegehahn (handelsüblich), bevorzugt mit Vorratsspritze (handelsüblich), stärker bevorzugt in der Größenordnung von 50, 60 oder 100 ml (handelsüblich), und Injektionsspritze, bevorzugt 1, 5, 10 ml (handelsüblich), mit Griffplatte (z.B. Spezialanfertigung).

Die Kits mit einer pharmazeutischen Zusammensetzung können die pharmazeutische Zusammensetzung als Konzentrat enthalten. Dann wird bevorzugt die Trägerlösung derart ausgewählt, dass die Trägerlösung zusammen mit dem Konzentrat eine pharmazeutische Zusammensetzung in einer der erfindungsgemäß beschriebenen Konzentrationen ergibt.

Bevorzugt werden die zusätzlichen geeigneten Vorrichtungen des Kits u.a. ausgewählt aus der Gruppe bestehend aus:
- einer Spezial-Spritzampulle (handelsüblich), die eine wie zuvor beschriebene pharmazeutische Zusammensetzung, ggf. als Konzentrat, enthält, noch stärker bevorzugt mit Lidocain als Lokalanästhetikum;
- einer Ringer-Lösung, z.B. von B. Braun Melsungen AG;
- einer Entnahme- und Zuspritzkanüle (Mini-Spike Plus^{®}) von B. Braun Melsungen AG;
- Einmal-Injektions-Kanülen 40 - 60 mm (Sterican^{®}) von B. Braun Melsungen AG;
- Einmal-Injektions-Kanülen 70 - 100 mm (TSK-SUPRA): TSK Laboratory, Japan;
- einer Haltevorrichtung (mit Befestigungsvorrichtung), insbesondere einer Halteklaue (mit Befestigungsvorrichtung) (Spezialanfertigung);
- einem druckstabilen Überleitungsschlauch (Perfusor^{®}) von B. Braun Melsungen AG;
- einem Dreiwegehahn (Discofix^{®}) von B. Braun Melsungen AG, mit einer Einmalspritze 5 ml (Onmifix^{®}) von B. Braun Melsungen AG und einer Einmalspritze 60 ml (Onmifix^{®}) von B. Braun Melsungen AG;

Alle obengenannten Produkte bestimmter Hersteller können selbstverständlich auch durch äquivalente Produkte anderer Hersteller ersetzt werden.

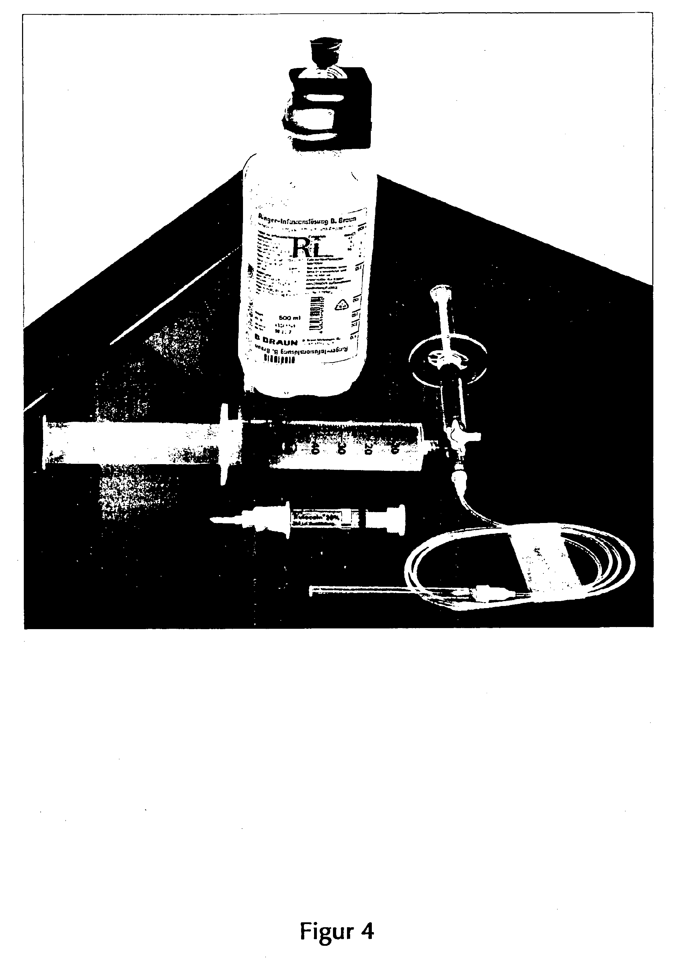

Eine bildliche Darstellung eines beispielhaften erfindungsgemäßen Kits ist in Figur 4 gezeigt. In Figur 1 wird eine beispielhafte Verabreichung unter Verwendung eines beispielhaften erfindungsgemäßen Kits gezeigt.

### Figurenbeschreibung:

- **Figur 1:**: stellt beispielhaft eine mögliche Verabreichung der erfindungsgemäß verwendeten niedrig-dosierten pharmazeutischen Zusammensetzung über ein erfindungsgemäßes Kit im Bereich der Brustwirbelsäule dar.

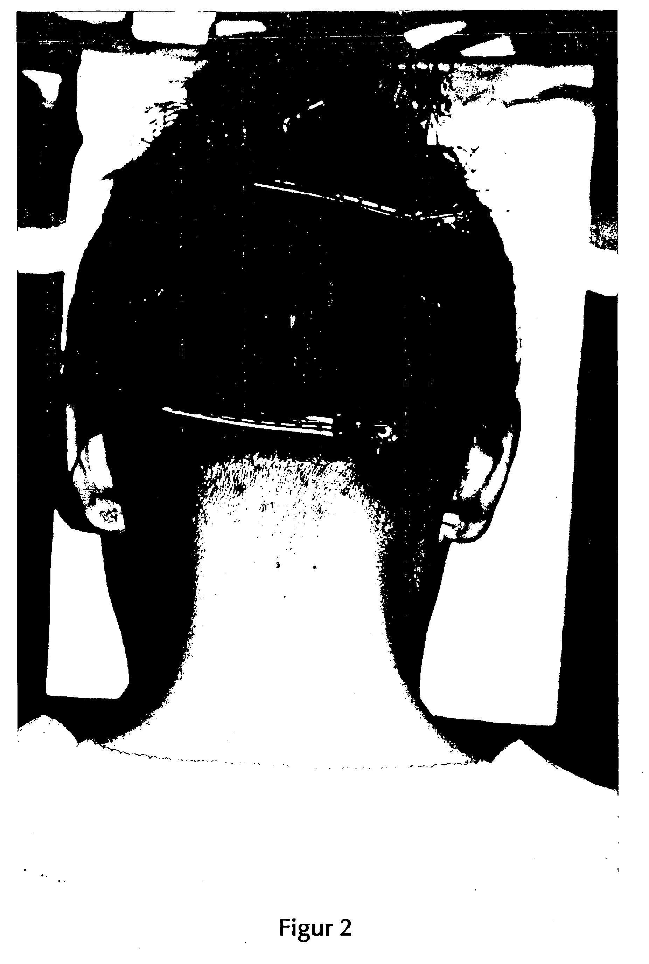

- **Figur 2:**: zeigt beispielhaft Einstichpunkte im Bereich der oberen und mittleren Halswirbelsäule.

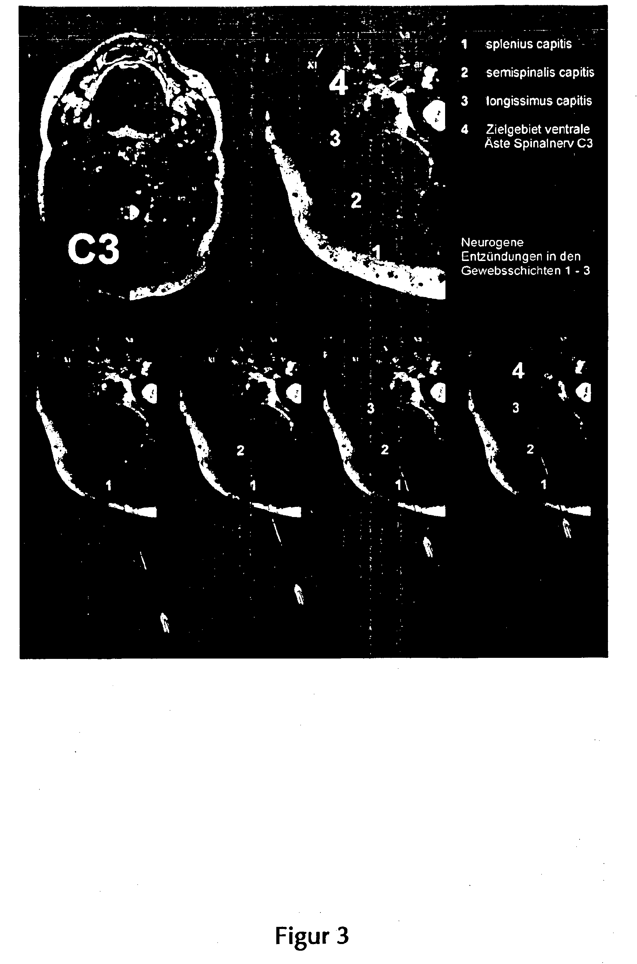

- **Figur 3:**: stellt schematisch eine vorliegend geeignete modifizierte Infiltrations-Anästhesie mit langsamem Nadelvorschub und gleichzeitigem, vor der Nadel ausgebrachtem schichtweisen Volumeneintrag (siehe untere Reihe, Schichten 1 bis 4) dar.
Obere und untere Reihe:
1 = splenus capitis ;
2 = semispinalis capitis ;
3 = longissimus capitis;
4 = Zielgebiet ventrale Äste des Spiralnervs C3;
Es sind neurogene Entzündungen in den Gewebsschichten 1 bis 3 zu sehen.
- **Figur 4:**: zeigt beispielhaft ein erfindungsgemäß verwendetes Kit, hier ein Spezial-Injektionskit zur Verabreichung der erfindungsgemäß verwendeten niedrig-dosierten pharmazeutischen Zusammensetzungen.

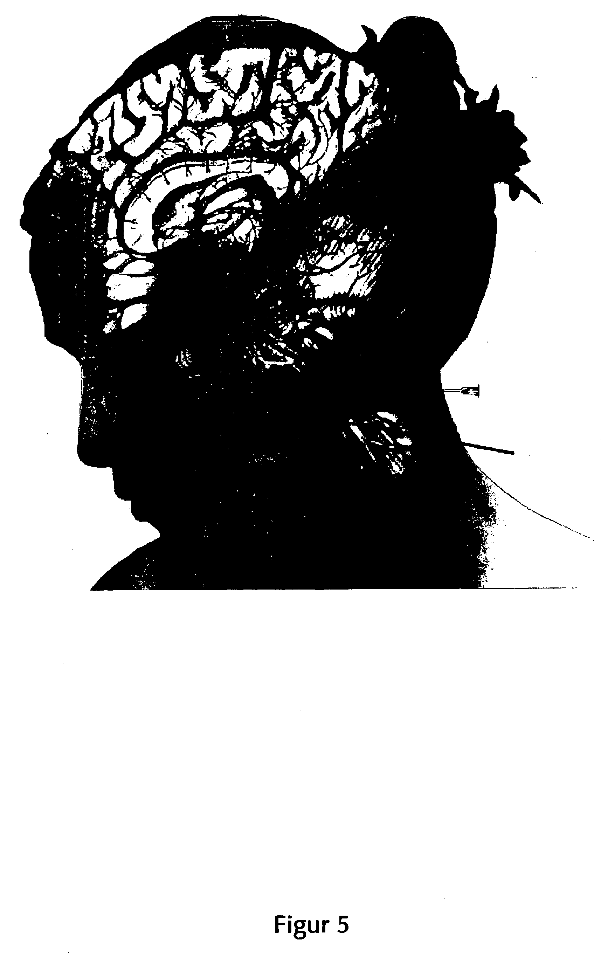

- **Figur 5:**: zeigt schematisch die Blockade des Ganglion cervicale superior (SCG), dessen postganglionäre Fasern die vordere, die mittlere und Teile der hinteren Hirnarterie innervieren.

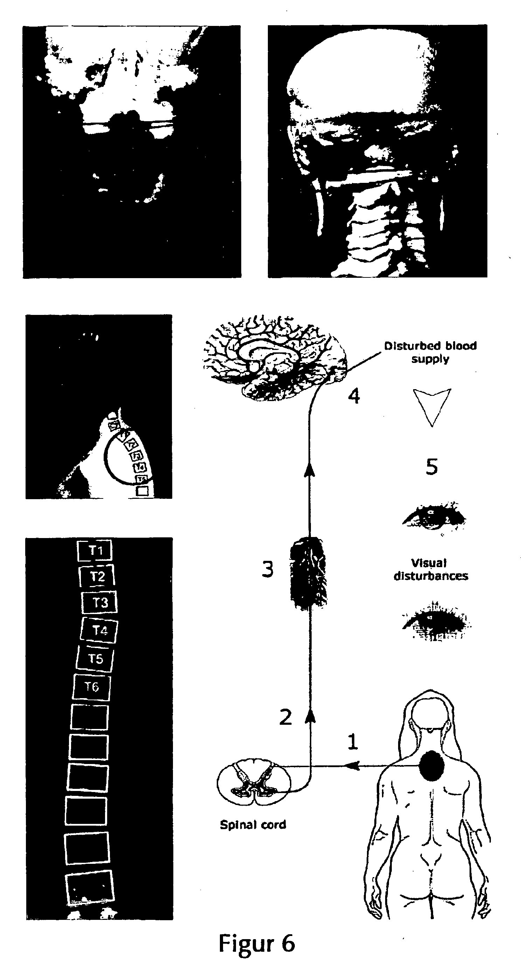

- **Figur 6:**: beschreibt Röntgenaufnahmen der oberen Halswirbelsäule vor und während eines Migräneanfalls (den ersten Halswirbel in der Regel geneigt und gleichzeitig gedreht oben links und oben rechts), meistens zur Seite der Migräne-5chmerzen (>70%). Am gleichseitigen Proc. transversus des Wirbels können regelmäßig starke Schmerzen provoziert werden. Zusätzlich weisen Migräne-Patienten oft funktionelle Veränderungen der oberen Brustwirbelsäule auf, die primär schmerzhaft sind oder in deren Bereich Schmerzen provoziert werden können (Abb. siehe links Mitte und links unten), von denen wir annehmen, dass sie die Symptome der Migräne-Aura wie z.B. Sehstörungen hervorrufen.

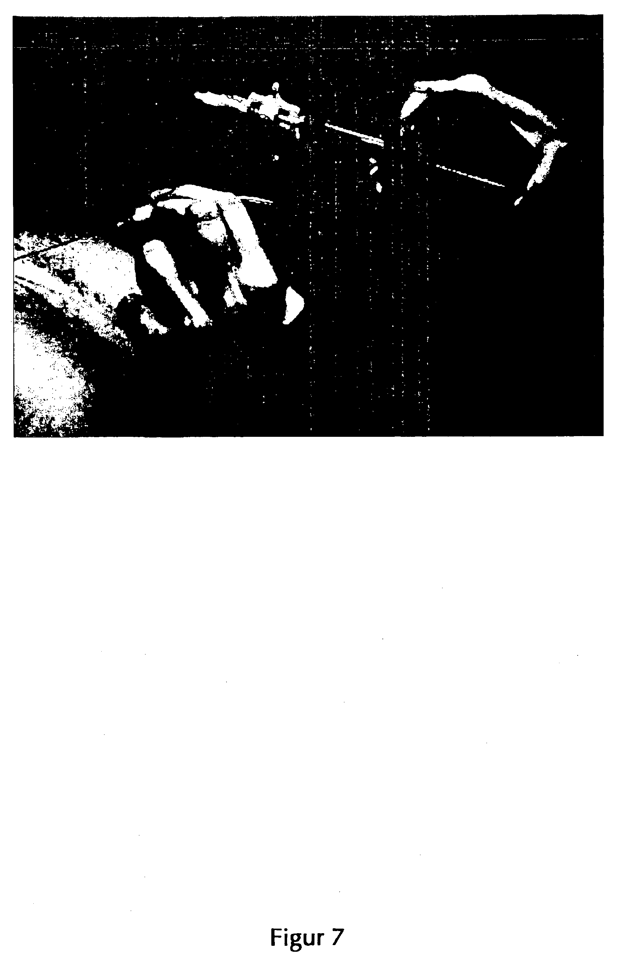

- **Figur 7:**: illustriert im Bild die erfindungsgemäße Verabreichung nach dem erfindungsgemäßen Verfahren der (modifizierten) Infiltrations-Anästhesie (unter Verwendung eines hier beschriebenen Kits).

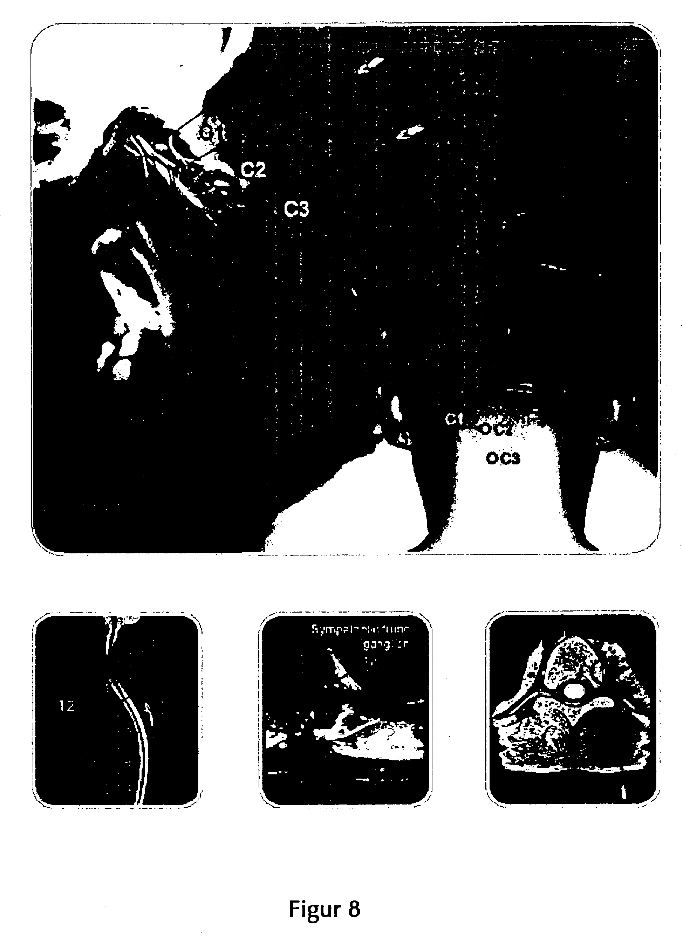

- **Figur 8:**: zeigt die Verabreichung des erfindungsgemäß eingesetzten niedrig-dosierten Lidocains und die Setzung von wiederholten Sympathicus-Blockaden, standardmäßig mittels tiefer dorsaler zerviko-thorakaler Infiltration mit niedrig-dosiertem Lidocain in Ringer-Lösung in einer Konzentration zwischen 0,025% und 0,2 % mit einem Volumen von 6 - 24 ml pro Infiltration. Zur Vermeidung von Gewebsverletzungen wurden die Kanülen grundsätzlich unter konstanter Flüssigkeitsabgabe in das Gewebe vorgeschoben (siehe unten, insbesondere unten rechts).

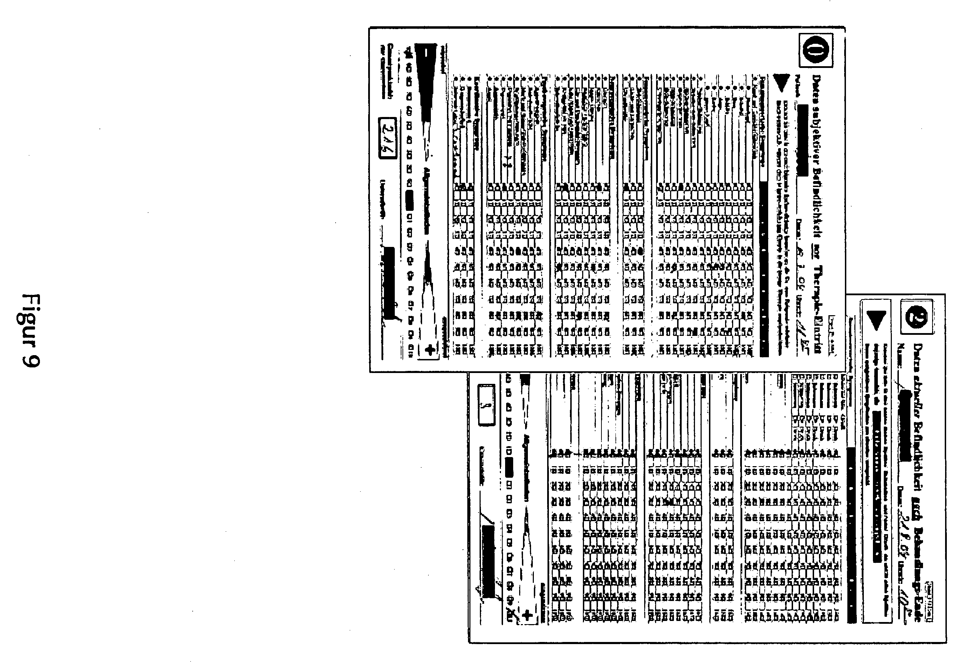

- **Figur 9:**: illustriert hier eingesetzte standardisierte Fragebögen auf der Basis von visuellen Analog-Skalen mit 10 Punkten für die höchste Intensität von Symptomen. Es wurde das Anfangsstadium vor Eintritt in die Behandlung erfasst und die Therapiewirkungen kontrolliert. Bogen 1 dokumentiert die Intensitäten aller Schmerzen und Beschwerden einer Patientin mit chronischer Migräne bei ihrem letzten schweren Anfall ohne Verwendung von Medikamenten. Bogen 2 zeigt das typische Ergebnis am Ende einer Behandlung, von der Patientin selbst ausgefüllt und unterschrieben (Name und Unterschrift geschwärzt).

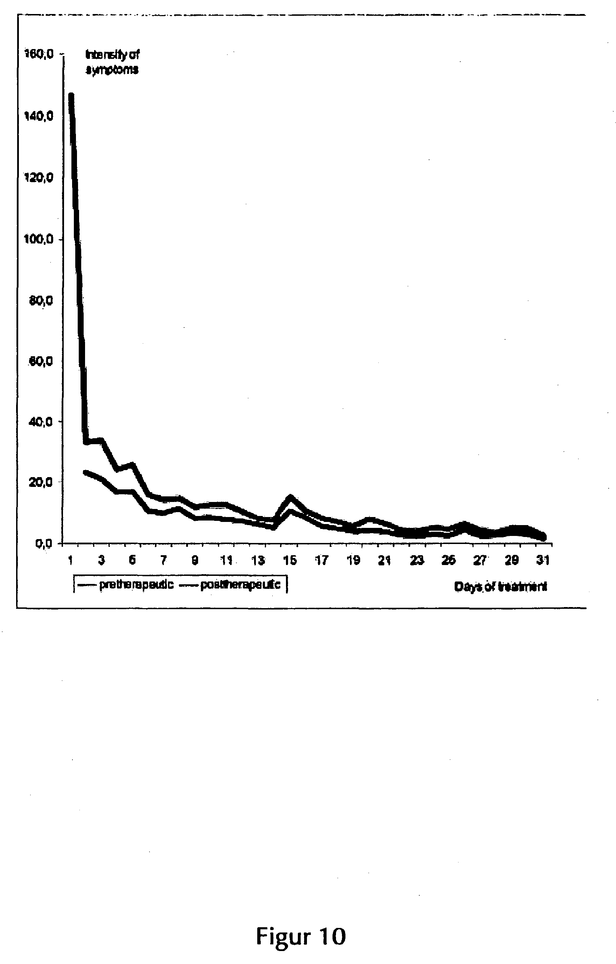

- **Figuren 10:**: ist eine Darstellung der täglich vor jeder Behandlung (pretherapeutic) bei Migräne und Kopfschmerzen vom Spannungs-Typ dokumentierten, patientenseitig bewerteten Intensitäten aller Symptome sowie der allgemeinen Befindlichkeit im Verlauf einer beispielsweise 5-wöchigen Behandlung.

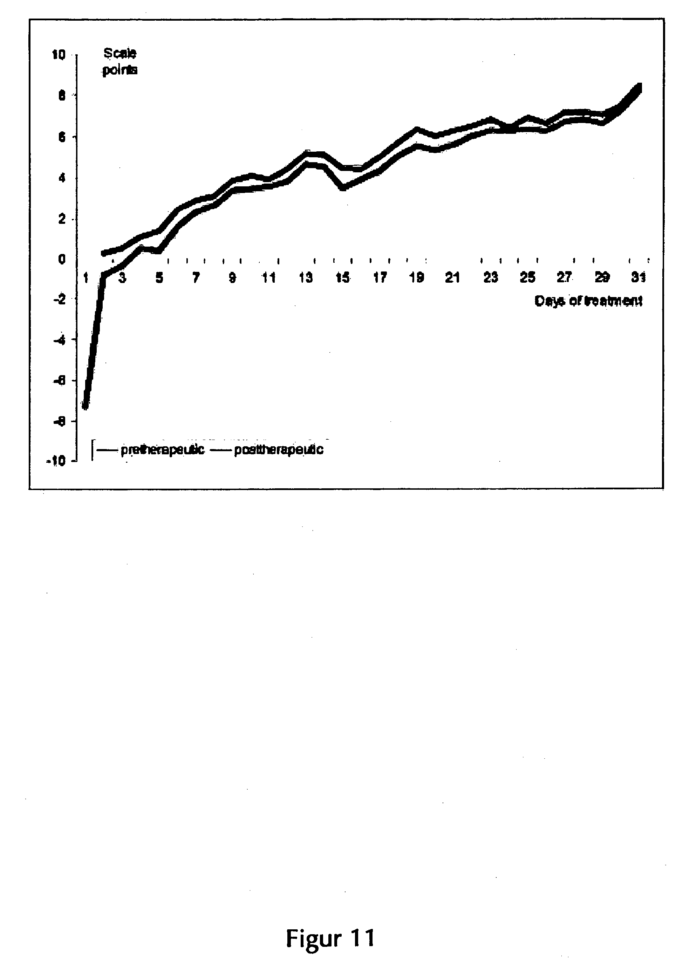

- **Figuren 11:**: ist eine Darstellung der täglich nach jeder Behandlung (posttherapeutic) bei Migräne und Kopfschmerzen vom Spannungs-Typ dokumentierten, patientenseitig bewerteten Intensitäten aller Symptome sowie der allgemeinen Befindlichkeit im Verlauf einer beispielsweise 5-wöchigen Behandlung.

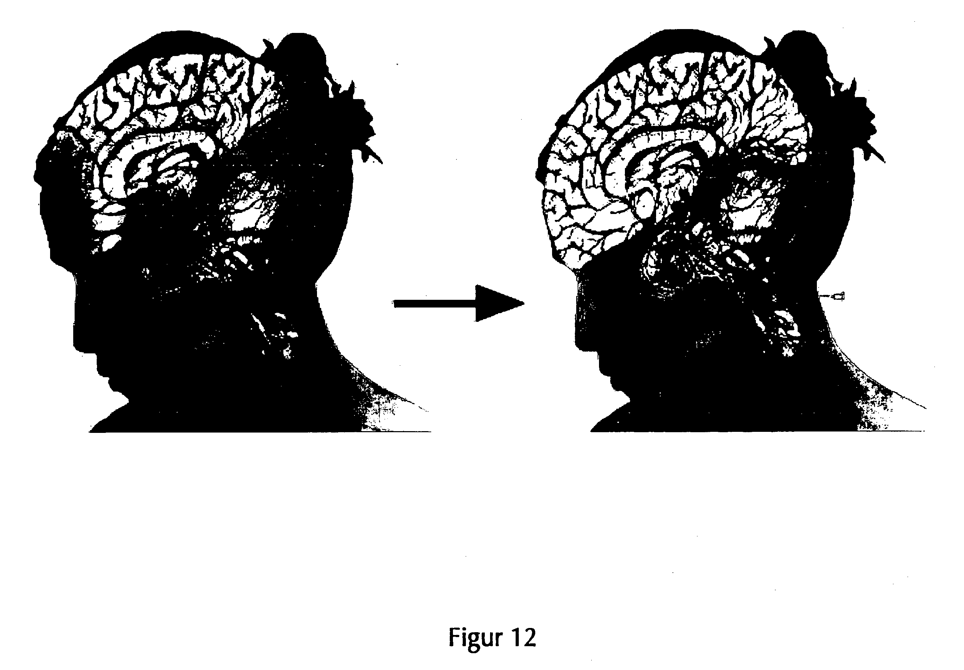

- **Figur 12:**: zeigt eine schematische Darstellung des erfindungsgemäß erreichten Therapieeffekts bei chronischen Migränikern durch wiederholte tiefe dorsale zerviko-thorakale Sympathicus-Blockaden, insbesondere die örtliche Hemmung der Nervenerregung und den anti-inflammatorischen Einfluss des Lidocains in tiefen zerviko-thorakalen sympathisch-sensorischen neuro-immunen und neuro-muskulären Trigger-Regionen von Migräne und Migräne-Aura.

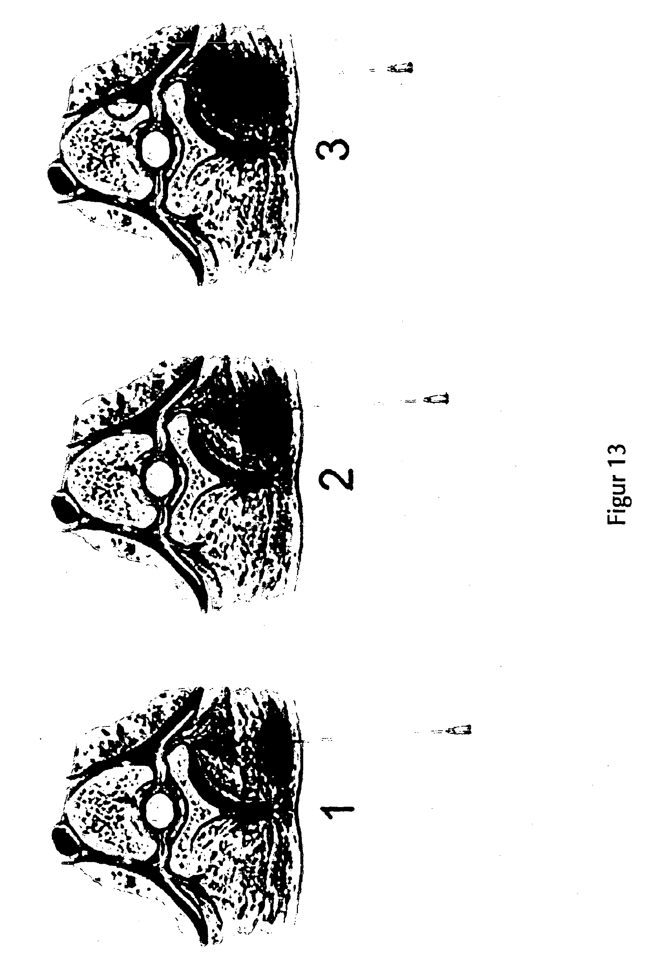

- **Figur 13:**: illustriert das erfindungsgemäße Verfahren der modifizierten Infiltrations-Anästhesie mit langsamem Nadelvorschub und gleichzeitigem, vor der Nadel ausgebrachtem schichtweisem Volumeneintrag (siehe Figur 13 in Teilfiguren 1-3, Schichten 1 bis 4). Nadelvorschub, Teilvolumina und Injektionsgeschwindigkeit richten sich nach der Gewebskonsistenz und Patientenangaben über Druck- und/oder Schmerz-Empfinden in einzelnen Gewebsschichten während des Nadelvorschubs unter Benützung einer 10-Punkte-Analog-Bewertung. Wegen der kurzfristigen Blockierung gewebsständiger Nozizeptoren, der patientenseitigen Kommentierungsmöglichkeit und der im Prinzip »stumpfen Präparation« der Gewebe durch die Injektions-Flüssigkeit und nicht primär durch die Nadel ist mit dieser Technik eine gewebsschonende und zugleich komfortable Anästhesie möglich. Sie schafft Spielraum für die in der Regel erforderliche Repetition am selben Ort. Die Patientenangaben über Druck und/oder Schmerz können zur Beurteilung z.B. von Intensität und Ausdehnung neurogener Entzündungen in unterschiedlichen Gewebs-Tiefen herangezogen werden. Dies erlaubt zugleich eine Beurteilung noch wirksamer Pathomechanismen und deren ggf. biomechanische Ursachen sowie die klinische Beurteilung des Heilungsverlaufs.

### Experimente/Behandlungsergebnisse

Die vorliegende Erfindung soll durch die beigefügten Beispiele näher erläutert werden, ohne jedoch auf diese Beispiele eingeschränkt zu sein.

### 1. Allgemeines

In allen folgenden Behandlungen wurde eine erfindungsgemäß verwendete pharmazeutische Zusammensetzung mit niedrig-dosiertem Lidocain in einer Konzentration von 0,1 Gew.-% in einer Ringerlösung verabreicht. Art und Umfang der beklagten Schmerzen und Beschwerden bestimmen unter (ausschließlich) neuroanatomischen und funktionspathologischen Gesichtspunkten die ursachenorientierte Auswahl von Injektionsort, Injektionsmenge und Injektionstiefe sowie die Auswahl der verwendeten Injektionskanülen, z.B. innerhalb einer Bandbreite zwischen 20 und 100 mm Länge, bei grundsätzlich identischer, jedoch gegenüber herkömmlicher Verfahrensweise grundlegend veränderter, Injektionstechnik (siehe z.B. Figuren 1 bis 3).

Vor Behandlungsbeginn werden mit Ausnahme von Benzodiazepinderivaten sämtliche "schmerz- und beschwerdespezifische" Medikamente spontan abgesetzt. Benzodiazepinderivate werden schleichend eliminiert.

### 2. Behandlung von Migräne mit niedrig-dosiertem Lidocain

Zur Behandlung von Migräne werden Patienten nach eingehender klinischer Untersuchung Gaben einer erfindungsgemäßen pharmazeutischen Zusammensetzung mit niedrig-dosiertem Lidocain (in einer Konzentration von 0,1 Gew.-%) lokal injiziert. Anzahl der Injektionen und Injektionsorte sind abhängig vom aktuellen Untersuchungsbefund sowie von Patientenangaben zu Schmerzen und Beschwerden. Bevorzugt werden tiefe paravertebrale Injektionen von dorsal in antero-lateraler Stichführung in Gewebe der oberen Halswirbelsäule (s. Figur 2) oder der oberen Brustwirbelsäule (s. Figur 1) vorgenommen, je Behandlungssitzung in der Regel 2 bis 4 Injektionen. Danach ist innerhalb weniger Minuten eine vollständige Anfallsdurchbrechung bei Patienten festzustellen; das heißt eine vollständige Schmerz- und Beschwerdefreiheit ohne individuell typische Rezidivneigung, wie sie speziell bei einer Migränedauer von mehreren Tagen unter systemischpharmakologischer Behandlung mit Serotonin-Agonisten (Triptane) und NSAR-Präparaten regelmäßig beobachtet wird.

Aktueller Fall:
Patient: 42-jähriger, international tätiger Fotograf und Journalist.
Vorgeschichte: Kopfschmerzen seit Kindesalter, zuletzt täglich ein bis zweimal Migräneschmerzen und -beschwerden; aus beruflichen Gründen immer mit Schmerzmitteln unterdrückt.
Behandlung: Medikamenten-Entzug innerhalb von einem Tag bei gleichzeitig einsetzender Injektionsbehandlung (niedrig-dosiertes Lidocain in einer Konzentration von 0,1 Gew.-%). (s. Figur 2: Einstichpunkte im Bereich der oberen und mittleren Halswirbelsäule)
Ergebnis: Der Patient war nach 2 ½ Behandlungstagen und seither dauerhaft schmerz- und beschwerdefrei.

### 3. Behandlung weiterer Schmerzen und Beschwerden mit niedrig-dosiertem Lidocain

### 3.1 Behandlung episodischer und chronischer Kopfschmerzen vom Spannungs-Typ:

Behandlung: Die Anzahl der Injektionen und Injektionsorte sind abhängig vom aktuellen Untersuchungsbefund sowie von Patientenangaben zu Schmerzen und Beschwerden. Bevorzugt werden tiefe paravertebrale Injektionen in Gewebe der oberen und mittleren Halswirbelsäule vorgenommen, je Behandlungssitzung in der Regel 6 bis 8 Injektionen mit gegenüber der vorbeschriebenen Migräne-Behandlung geringerer Einstichtiefe.

Ergebnis: In einem dafür typischen Behandlungsfall folgte der Injektionsserie eine spontane, anfangs ca. 2 bis 4 Stunden anhaltende Schmerzfreiheit ohne die von Injektionen mit herkömmlicher Lokalanästhetika-Konzentration bekannten Sensibilitätsstörungen. Eine Repetition der Behandlung führte zur schrittweisen Verlängerung der schmerzfreien Intervalle und schließlich zur dauerhaften Schmerzfreiheit.

### 3.2 Behandlung therapieresistenter chronischer Schulter-Arm-Schmerzen

Behandlung: 2 bis 4 tiefe, seitengleiche paravertebrale Injektionen in Gewebe der mittleren Halswirbelsäule.

Ergebnis: Spontane Remission von Schmerzen und Beschwerden.

### 3.3 Behandlung therapieresistenter chronischer Sensibilitätsstörungen an Armen und Händen mit Verlust der rohen Kraft

Behandlung: 2 bis 4 tiefe, seitengleiche paravertebrale Injektionen in Gewebe der mittleren bzw. unteren Halswirbelsäule.

Ergebnis: Spontane Remission der Kraftlosigkeit; Sensibilitätsstörungen remittierten i.d.R. vollständig nach zwei bis vier weiteren identisch reproduzierten Behandlungssitzungen.

### 3.4 Behandlung therapieresistenter chronischer Schmerzen der Hüften, Beine und Füße (chronisches LWS-Syndrom) mit oder ohne Sensibilitätsstörungen

Behandlung: 2 bis 4 tiefe, seitengleiche paravertebrale Injektionen in Gewebe der mittleren und unteren Lendenwirbelsäule bzw. der oberen Kreuzbeinregion (: L3 bis SI alternierend).

Ergebnis: Vollständige Remission altersabhängig i.d.R. innerhalb von 6 bis 10 Behandlungssitzungen.

Aktueller Fall:
71-jähriger Patient. Seit 10 Jahren progrediente Krankheitsentwicklung eines chronischen, linksseitigen LWS-Syndroms mit belastungsabhängigen Schmerzen und
Taubheitsgefühl in Oberschenkel und Knie sowie belastungsabhängigem, progredientem Kraftverlust des linken Beines.
Behandlung: Repetitive paravertebrale Injektionen L3 bis L5 links.
Ergebnis: Vollständige Remission aller Beschwerden nach 10 Behandlungssitzungen.

### 3.5 Behandlung schwerer (chronischer) therapieresistenter Menstruationsbeschwerden (Menorrhagien)

Behandlung: Tiefe doppelseitige paravertebrale Injektionen in Gewebe der oberen Lendenwirbelsäule (L2).

Ergebnis: Spontane Remission der Unterbauchschmerzen und -beschwerden; i.d.R. vollständige Normalisierung von Blutungsmenge und Blutungsdauer nach zwei bis vier weiteren, identisch reproduzierten Behandlungssitzungen.

### 3.6 Behandlung von (chronisch auftretenden) Schlafstörungen ohne psychovegetativem Hintergrund

Behandlung: Tiefe doppelseitige paravertebrale Injektionen in Gewebe der mittleren Brustwirbelsäule (Infiltrations-Anästhesien oberflächlicher und tiefer Weichteilstrukturen mit abschließend simultaner Blockade der thorakalen Grenzstrang-Ganglien T4).

Ergebnis: Schrittweise Remission der Schlafstörung i.d.R. innerhalb von 8 bis 10 Behandlungssitzungen.

### 3.7 Behandlung von (chronisch auftretenden) Schlafstörungen mit psychovegetativem Hintergrund

Behandlung: Tiefe linksseitige paravertebrale Injektionen in Gewebe der mittleren Brustwirbelsäule (Infiltrations-Anästhesien oberflächlicher und tiefer Weichteilstrukturen mit abschließend simultaner Blockade der thorakalen Grenzstrang-Ganglien T4 bis T6).

Ergebnis: Schrittweise Remission der Schlafstörung i.d.R. innerhalb von 8 bis 12 Behandlungssitzungen.

### 3.8 Behandlung chronisch rezidivierender Zustände depressiver Verstimmung sowie leichter bis mittelschwerer Depressionen ohne aggressive Komponente

Behandlung: Tiefe linksseitige paravertebrale Injektionen in Gewebe der mittleren Brustwirbelsäule (Infiltrations-Anästhesien oberflächlicher und tiefer Weichteilstrukturen mit abschließend simultaner Blockade der thorakalen Grenzstrang-Ganglien T5 und T6).

Ergebnis: Schrittweise Remission der Befindlichkeitsstörungen i.d.R. innerhalb von 8 bis 10 Behandlungssitzungen.

### 3.9 Behandlung chronisch rezidivierender Zustände depressiver Verstimmung sowie leichter bis mittelschwerer Depressionen mit aggressiver Komponente

Behandlung: Tiefe linksseitige paravertebrale Injektionen in Gewebe der mittleren Brustwirbelsäule (Infiltrations-Anästhesien oberflächlicher und tiefer Weichteilstrukturen mit abschließend simultaner Blockade der thorakalen Grenzstrang-Ganglien T4 bis T6).

Ergebnis: Schrittweise Remission der Befindlichkeitsstörungen i.d.R. innerhalb von 8 bis 12 Behandlungssitzungen.

### 4. Behandlung von Neuralgien

### 4.1 Behandlung der Trigeminusneuralgie

Behandlung: Oberflächliche, großflächig ausgebrachte Injektion des betroffenen Nervenastes am Ort seiner stärksten Irritation;

Ergebnis: i.d.R. Spontan-Remission. Selten erforderlich: ein bis zwei Wiederholungs-Injektionen.

### 4.2 Behandlung der Occipitalisneuralgie

Behandlung: Je nach Untersuchungsbefund oberflächliche, großflächig ausgebrachte oder tiefe Injektionen des bzw. der betroffenen Nervenäste;

Ergebnis: oberflächliche Nervenäste reagieren i.d.R. mit Spontan-Remission. Selten erforderlich: ein bis zwei Wiederholungsinjektionen. Tiefe Nervenäste reagieren i.d.R. eher verzögert, so dass Wiederholungsinjektionen hierbei notwendig werden.

### 4.3 Behandlung der therapieresistenten (chronischen) postzosterischen Thorakal- bzw. Lumbal-Neuralgie

Behandlung: Tiefe gleichseitige paravertebrale Injektionen in Gewebe der betroffenen Thorakal- bzw. Lumbal-Segmente (Infiltrations-Anästhesien oberflächlicher und tiefer Weichteilstrukturen mit abschließend simultaner Blockade der betroffenen thorakalen bzw. lumbalen Grenzstrang-Ganglien).

Ergebnis: Schrittweise Remission der neuralgischen Schmerzzustände altersabhängig innerhalb von 2 bis 4 Wochen bei täglichen, ggf. zweimal täglichen Injektionen.

### 4. Behandlung von Verletzungen

### 4.1 Behandlung akuter kleinflächiger Verletzungen, soweit keine Operationsbedürftigkeit besteht

Behandlung: Je nach Untersuchungsbefund gezielte oberflächliche Injektionen in die traumatisierten Gewebe mit der vorstehend beschriebenen Injektionsmethode des langsamen Kanülenvorschubs bei gleichzeitigem, vor der Kanüle ausgebrachtem Flüssigkeitseintrag.

Ergebnis: spontane Schmerzfreiheit ohne Sensibilitätsstörungen. Ein- bis zweimalige Repetition in 24-stündigen Abständen genügt i.d.R. zur Halbierung der sonst üblichen (Wund-)Heilungsdauer.

### 4.2 Behandlung chronischer Verletzungen

Behandlung: Abhängig vom klinischen Untersuchungsbefund gezielte Injektionen in die traumatisierten Gewebe mit der vorstehend beschriebenen Injektionsmethode. Ergebnis: spontane Schmerzreduktion ohne Sensibilitätsstörungen. Mehrfache Repetition in 24-stündigen Abständen führt zur schrittweisen und i.d.R. vollständigen Remission. Die Anzahl der Injektionen pro Sitzung sowie der Repetitionen ist abhängig von Art und Ausdehnung der traumatisierten Gewebe, von Art und Umfang ihrer statischen und dynamischen Belastung sowie vorrangig vom Lebensalter der Patienten.

### 5. Weitere Beispiele der modifizierten Infiltrations-Anästhesie bei Migräne

### 5.1. Im vorliegenden Experiment wurden Migränepatienten erfindungsgemäß mit niedrig-dosiertem Lidocain therapiert.

5.1.1. Migräne und ihre Begleitsymptome sind durch sensorische, autonome (sympathisch/parasympathische) sowie verschiedene intrazerebrale neurovaskuläre Veränderungen gekennzeichnet. Bekanntermaßen kann die Verabreichung von β-Blockern Migräne-Anfälle verhindern bzw. reduzieren. Dies deutet auf eine ursächliche Beteiligung des sympathischen Nervensystems (SNS) an der komplexen Pathophysiologie der Migräne. Das SNS spielt auch eine wichtige Rolle bei neuropathischen Schmerzen durch sympathisch-sensorische neuro-immune Wechselwirkungen. Migräne besitzt Merkmale neuropathischer Schmerzen; Medikamente gegen Migräne können gegen neuropathische Schmerzen wirksam sein. Ferner wirkt die Stimulation der Nn. occipitalis der Migräne und anderen Kopfschmerzen entgegen. Lidocain hemmt die durch NFkB vermittelte Produktion von Zytokinen aus Entzündungszellen.

Vor diesem Hintergrund erscheint es sinnvoll, die Migräne assoziierte Fehlfunktion des SNS durch dorsale Sympathicus-Blockaden (siehe Fig. 5) zu behandeln, um damit Migräne, Migräne-Aura und ihre Begleitsymptome zu lindern.

Röntgenaufnahmen der oberen Halswirbelsäule zeigen vor und während eines Migräneanfalls den ersten Halswirbel in der Regel geneigt und gleichzeitig gedreht (siehe Fig. 6), meistens zur Seite der Migräne-Schmerzen (>70%). Am gleichseitigen Proc. transversus des Wirbels können regelmäßig starke Schmerzen provoziert werden. Wir nehmen an, dass diese Schmerzen aufgrund örtlicher Entzündungen entstehen infolge der Wirbeldistorsion mit Muskelverspannungen, die sensorischsympathische neuroimmune Reaktionen nach sich ziehen.

Zusätzlich weisen Migräne-Patienten oft funktionelle Veränderungen der oberen Brustwirbelsäule auf, die primär schmerzhaft sind oder in deren Bereich Schmerzen provoziert werden können (siehe Fig. 6), von denen wir annehmen, dass sie die Symptome der Migräne-Aura wie z.B. Sehstörungen hervorrufen (siehe Fig. 6).

Wir zeigen hier, dass Sympathicus-Blockaden mit dem erfindungsgemäß verwendeten niedrig-dosierten Lidocain wirksam bei der Behandlung von Migräne und Migräne-Aura sind und zusammen mit speziellen Anweisungen für Migräne-Patienten zur Verbesserung ihrer statischen und dynamischen Körperhaltung und ihrer allgemeinen Lebensführung sowie einem lang anhaltenden therapeutischen Effekt von mindestens einigen Jahren nach Ende der jeweiligen Behandlungsperiode führen.

### 5.1.2. Versuchsanordnung

Unter Anwendung der diagnostischen Kriterien der IHS (2003) wurden dazu 84 Patienten mit chronischer Migräne in eine retrospektive Analyse einbezogen. 69 Patienten (81%) waren weiblich, 15 (19%) waren männlich. Das Durchschnittsalter betrug 46,1 Jahre, das Intervall 18 - 76 Jahre. 43 Patienten (51% von allen) litten an Migräne, 31 (37% von allen) litten an Migräne mit Aura, 53 Patienten (63% von allen) litten an Migräne ohne Aura. Die durchschnittliche Krankheitsdauer betrug 22,9 Jahre (2 - 62) mit 7,4 Migräne-Anfällen (3 - 10) pro Monat mit einer durchschnittlichen Dauer von 52,0 Stunden (2 - 216) und einer Schmerz-Intensität auf dem Höhepunkt eines Anfalls von durchschnittlich 8,5 Punkten innerhalb einer IO-Punkte-Skala. 41 Patienten (49% von allen) litten sowohl an Migräne als auch an Kopfschmerzen vom Spannungstyp.

Zunächst wurden erfindungsgemäß wiederholte Sympathicus-Blockaden mittels tiefer dorsaler zerviko-thorakaler Infiltration (Abb. 6 - 9) mit niedrig dosiertem Lidocain in Ringer-Lösung in einer Konzentration zwischen 0,025% und 0,2 % mit einem Volumen von 6 - 24 ml pro Infiltration gesetzt. Zur Vermeidung von Gewebsverletzungen wurden die Kanülen grundsätzlich unter konstanter Flüssigkeitsabgabe in das Gewebe vorgeschoben (siehe Figur 5 und 6).

Die Patienten wurden innerhalb von 4 (n=21), 5 (n=43) und 6 (n=20) Wochen täglich morgens und abends während einer Migräne-Aura und eines Migräneanfalls sowie während der anfallsfreien Perioden behandelt ohne Verwendung anderer Medikamente. Vorangegangene Medikation wurde vor Behandlungsbeginn abgesetzt. Mit einem standardisierten Fragebogen auf der Basis von visuellen Analog-Skalen mit 10 Punkten für die höchste Intensität von Symptomen (siehe Figur 7) wurde das Anfangsstadium vor Eintritt in die Behandlung erfasst und die Therapiewirkungen kontrolliert.

Insgesamt wurden 36 Migräne-Schmerzen und Migräne-Begleitsymptome vor und nach jeder Behandlung zweimal pro Tag dokumentiert (prä- und posttherapeutisch) zur engmaschigen Kontrolle des Behandlungsverlaufs während der gesamten Behandlungs-Periode. Diese Daten wurden mit den Ergebnissen des formal identischen Bogens "0" verglichen, den Patienten vor Beginn ihrer Behandlung ausfüllen mussten und der ihren letzten schweren Migräneanfall ohne Einsatz von Medikamenten abbildete. Die Veränderung der Summe aller Schmerz-Intensitäten und Migräne-Begleitsymptome diente als Maßstab für die Wirksamkeit der Behandlung zusammen mit dem Rückgang der Kopfschmerz-Frequenz und der Migräne-Anfälle.

### 5.1.3. Ergebnis:

Die Gesamtsummen aller Schmerz-Intensitäten und Migräne-Begleitsymptome zeigten unter der Behandlung einen übereinstimmenden Rückgang in allen Behandlungs-Perioden von 4, 5 oder 6 Wochen auf weniger als 10% am Ende der ersten Woche (siehe Figur 8) und eine entsprechend positive Zunahme des Allgemeinbefindens (siehe Figur 9).

Die 4-wöchige Behandlungs-Gruppe erreichte am Ende einen Rückgang aller Schmerzen und Begleitsymptome auf 3,2%, die 5 bzw. 6 Wochen lang behandelten Gruppen erreichten bei ähnlichen Verläufen einen Rückgang auf 1,1% beziehungsweise 1,0%. Migräne-Aura und Migräne-Anfälle konnten regelmäßig innerhalb von 5 bis 10 Minuten nach den Infiltrationen durchbrochen werden.

Kopfschmerzen vom Spannungs-Typ wurden gleichzeitig schrittweise bis zum Ende jeder Behandlungs-Periode beseitigt.

Diese Ergebnisse zeigen die hohe Wirksamkeit unserer neuen Technik der wiederholten tiefen dorsalen zerviko-thorakalen Sympathicus-Blockaden bei chronischen Migränikern, die gegenüber konventionellen Pharmakotherapien resistent sind. Die dem Therapieerfolg zugrunde liegenden Mechanismen umfassen
I.) die Rückgewinnung des gestörten Gleichgewichts der komplexen Einstellungen sympathischer CoTransmitter einschließlich von Prostaglandinen, ATP, Adenosin, NO und verschiedener Neuropeptide;
II.) die Neueinstellung der gestörten langschleifigen sympathisch-trigeminalen und sympathisch-parasympathischen Wechselbeziehungen;
III.) die örtliche Hemmung der Nervenerregung und den anti-inflammatorischen Einfluss des Lidocain in tiefen zerviko-thorakalen sympathisch-sensorischen neuro-immunen und neuro-muskulären Trigger-Regionen
von Migräne und Migräne-Aura (siehe Figur 10).

### 5.2 Vergleichsversuche mit Lidocain, Mepivacain und Bupivacain

### 5.2.1. Materialien

In einem Vergleichsversuch wurden Lidocain, Mepivacain und Bupivacain in ihrer Wirksamkeit miteinander verglichen. Von allen getesteten und hier genannten Lokalanästhetika weist dabei unter den klinischen Gesichtspunkten seiner anästhetischen Wirksamkeit die geringst mögliche Nebenwirkung auf. Dabei zeigen andere Lokalanästhetika in höherer Konzentration eine längere Wirksamkeit. So zeigten die getesteten Lokalanästhetika im klinischen Versuch in äquipotenter Verdünnung 1:5 (gegenüber der empfohlenen Konzentration für den sensiblen Block) eine zu Lidocain vergleichbare Wirkung.

Verglichen wurden:
- Lidocain (sensibler Block in 0,1%-iger Konzentration (Endkonzentration der Formulierung): Wirkungsdauer 1 - 3 Stunden) ***
- Mepivacain (sensibler Block in 0,1%-iger Konzentration (Endkonzentration der Formulierung): Wirkungsdauer 2 - 3 Stunden) ***
- Bupivacain (sensibler Block in 0,05%-iger Konzentration (Endkonzentration der Formulierung): Wirkungsdauer 4 - 6 Stunden) ***
   *** Quelle:
   Ammon, HPT: Arzneimittelneben- und wechselwirkungen.
   Ein Handbuch und Tabellenwerk für Ärzte und Apotheker (1991) Versuchsanordnung:

### 5.2.2. Versuchsanordnung

Getestet wurden 3 männliche Patienten mit Kopfschmerzen vom Spannungstyp, (Alter 36, 38, 43). Die Patienten wurden wegen Nacken-, Stirn- und Schläfenschmerzen infolge ventralflektorischer Insuffizienz der oberen HWS eine Woche nach Absetzen aller systemisch wirksamer Schmerzmittel (NSAR-Präparate) je 7 Tage lang mit Prüf-Lokalanästhetika in äquipotenter Dosierung behandelt.

Dazu wurden tiefe Infiltrations-Anästhesien der Zervikal-Etagen C1 bis C3 vorgenommen nach Ziffer 490 GOÄ (Infiltrations-Anästhesien kleiner Bezirke im Bereich der Nervenwurzeln C1 bis C3).

Die Patienten erhielten entweder Lidocain oder Bupicacain oder Mepivacain in äquipotenter Verdünnung 1:5 (Endkonzentrationen der Formulierung 0,1 Gew.-%, 0,05 Gew.-%, bzw. 0,1 Gew.-%, s.o.) gegenüber den üblichen Konzentrationen für den sensiblen Block (0,5 Gew.-%, 0,25 Gew.-%, bzw. 0,5 Gew.-%).

Keiner der Patienten wusste, welches Lokalanästhetikum bei ihm verwendet wurde.

Die jeweiligen Schmerzintensitäten wurden in einer 10-Punkte-Skala der subjektiven Befindlichkeit patientenseitig dokumentiert jeweils vor Behandlungsbeginn und 30 Minuten nach Behandlungsende.

Die Infiltrations-Anästhesien wurden morgens 8.00 Uhr und abends 18.00 Uhr vorgenommen.

Verglichen wurden Schmerzverläufe aller drei Schmerz-Projektionsorte in einem Intensitäts-/Zeit-Diagramm über 7 Tage.

### 5.2.3. Ergebnis:

Unter normalen Bedingungen zeigten das langwirksame Bupivacain sowie das mittellang wirksame Mepivacain in äquipotenter Verdünnung gegenüber Lidocain eine etwa vergleichbare Wirkung und Therapieeffekt. Dabei zeigten die Schmerzintensitäts-Kurven der Patienten innerhalb der Beobachtungsperiode praktisch identische Verläufe.

### Vorteile der vorliegenden Erfindung

Die vorliegende Erfindung stellt die Verwendung von niedrig-dosiertem Lidocain oder eines 3-OH- oder 4-OH-Derivats davon bereit, in einer Konzentration von 0,05 Gew.-% bis 0,2 Gew.-%, zur Behandlung oder Prophylaxe von chronischen Erkrankungen oder (chronischen) Kopfschmerzerkrankungen, insbesondere der Therapie von chronischen bzw. wiederkehrend auftretenden Schmerzen, vor allem Kopfschmerzen, z.B. bei Migräne mit und ohne Aura, retinaler Migräne, chronischer Migräne, Kopfschmerzen vom Spannungstyp, anderen primären (auch juvenilen) Kopfschmerz-Syndromen, oder sonstigen (chronisch) auftretenden (Kopf)schmerzerkrankungen. Die Erfindung beschreibt des weiteren pharmazeutische Zusammensetzungen. Das niedrig-dosierte Lidocain oder ein 3-OH- oder 4-OH-Derivat davon ermöglichen die effektive Behandlung und Prophylaxe vieler chronischer krankhafter Zustände, insbesondere Schmerzzustände, ohne die von bisherigen Anwendungen bekannten und unerwünschten Nebenwirkungen. Dies betrifft insbesondere z.B. die Vermeidung der aufgrund der hohen Konzentration und Wirkdauer bisher eingesetzter Lokalanästhetika mögliche erhebliche Schädigung der Zellatmung, die zum partiellen Zelltod oder in besonderen Ausnahmefällen zum Organtod und damit zum Tod des Patienten führen kann. Erfindungsgemäß verwendete pharmazeutische Zusammensetzungen können besonders vorteilhaft auch zur Behandlung oder Prophylaxe von chronischen Schmerzerkrankungen wie bspw. Migräne, zur effektiven Schmerzausschaltung bei medizinischen Prozeduren oder vor allem bei Spätfolgen, vor allem bei Spätstadien chronischer Erkrankungen eingesetzt werden, die durch chronisch rezidivierende oder perpetuierende, häufig multifokale Entzündungen gekennzeichnet sind und damit auch Nerven schädigen können.

## Patentansprüche

1. Lidocain oder ein 3-OH oder 4-OH-Derivat davon in einer Konzentration von 0,05 Gew.-% bis 0,2 Gew.-% zur Verwendung in der Therapie oder Prophylaxe von chronischen Schmerz- oder Kopfschmerzerkrankungen durch Infiltrations-Anästhesie.

2. Lidocain oder ein 3-OH oder 4-OH-Derivat davon gemäß Anspruch 1 zur Verwendung gemäß Anspruch 1, **dadurch gekennzeichnet, dass** die chronische Kopfschmerzerkrankung eine primäre Kopfschmerzerkrankung, bevorzugt Migräne, ist.

3. Lidocain oder ein 3-OH oder 4-OH-Derivat davon gemäß einem der Ansprüche 1 oder 2 zur Verwendung gemäß Anspruch 1 oder 2, **dadurch gekennzeichnet, dass** das niedrig-dosierte Lidocain oder ein 3-OH oder 4-OH-Derivat davon in einer Konzentration von 0,05 Gew.-% bis 0,1 Gew.-%, in einer Konzentration von 0,05 Gew.-% bis 0,09 Gew.-% oder in einer Konzentration von 0,05 Gew.-% bis 0,08 Gew.-% eingesetzt wird.

4. Lidocain oder ein 3-OH oder 4-OH-Derivat davon gemäß einem der Ansprüche 1 bis 3 zur Verwendung gemäß einem der Ansprüche 1 bis 3, **dadurch gekennzeichnet, dass** die Infiltrations-Anästhesie durch Injektion im Bereich der Halswirbelsäule, der Nackenmuskulatur, des kranio-zervikalen Übergangs (Genick), der oberen, mittleren oder unteren Brustwirbelsäule, z.B. im Bereich der Thorakalsegmente/Grenzstrangganglien T1, T2, T3, T4, T5, T6, T7, T8, T9, T10, T11, oder T12 (auch genannt Th1, Th2, Th3, Th4, Th5, Th6, Th7, Th8, Th9, Th10, Th11, oder Th12), im Bereich der Lumbalsegmente L1, L2, L3, L4 oder L5, an der Schädelbasis (ventrale Äste von C1) sowie der ventralen Äste der Spinal-Nerven C2 C3, C4, C5, C6, C7, C8, im Bereich der Hirnarterien, der Nervenstränge in diesen Bereichen, der im Bereich der Nackenmuskulatur, der Thorakalsegmente T1 bis T3, des kranio-zervikalen Übergangs (Genick), im Bereich des Ganglia cervicale superius und stellatum, des splenus capitis, des semispinalis capitis, des longissimus capitis, sowie an der Schädelbasis (ventrale Äste von C1) oder der ventralen Äste der Spinal-Nerven C2 und C3 erfolgt.

5. Lidocain oder ein 3-OH oder 4-OH-Derivat davon gemäß einem der Ansprüche 1 bis 4 zur Verwendung gemäß einem der Ansprüche 1 bis 4, **dadurch gekennzeichnet, dass** das niedrig-dosierte Lidocain oder ein 3-OH oder 4-OH-Derivat davon zusätzlich mindestens ein Penetrationsmittel bzw. einen Penetrationsverstärker enthält.

6. Lidocain oder ein 3-OH oder 4-OH-Derivat davon gemäß Anspruch 5 zur Verwendung gemäß Anspruch 5, **dadurch gekennzeichnet, dass** das Penetrationsmittel bzw. Penetrationsverstärker ausgewählt wird aus Benzylalkhol, (2-(2-Ethoxy)ethanol, Propylenglykol, Methylpyrrolidon oder Dimethylsulfoxid (DMSO).

7. Lidocain oder ein 3-OH oder 4-OH-Derivat davon gemäß einem der Ansprüche 1 bis 6 zur Verwendung gemäß einem der Ansprüche 1 bis 6, **dadurch gekennzeichnet, dass** die pharmazeutische Zusammensetzung einmalig oder mehrmalig pro Tag, pro Woche oder pro Monat verabreicht wird.

8. Lidocain oder ein 3-OH oder 4-OH-Derivat davon gemäß einem der Ansprüche 1 bis 7 zur Verwendung gemäß einem der Ansprüche 1 bis 7, **dadurch gekennzeichnet, dass** die pharmazeutische Zusammensetzung 2-5 mal, 5-10 mal, 10 bis 20 mal, oder 20 bis 40 mal pro Tag, pro Woche, pro Monat oder pro Jahr verabreicht wird.

9. Lidocain oder ein 3-OH oder 4-OH-Derivat davon gemäß einem der Ansprüche 1 bis 8 zur Verwendung gemäß einem der Ansprüche 1 bis 8, **dadurch gekennzeichnet, dass** die chronischen Kopfschmerzerkrankungen ausgewählt werden aus der Gruppe umfassend primäre Kopfschmerzerkrankungen, umfassend Migräne, Migräne mit und ohne Aura, retinale Migräne, chronische Migräne, Kopfschmerzen vom Spannungstyp, Clusterkopfschmerz, andere trigemino-autonome Kopfschmerzerkrankungen und andere primäre (ggf. juvenile) Kopfschmerzen; oder sekundäre Kopfschmerzerkrankungen, umfassend Kopfschmerzen, die auf Kopf- und/oder Halswirbelsäulen (Hws) -Trauma zurückzuführen sind, Kopfschmerzen, die auf Gefäßstörungen im Bereich des Kopfes oder des Halses zurückzuführen sind, Kopfschmerzen, die auf nicht-vaskuläre intrakraniale Störungen zurückzuführen sind, Kopfschmerzen, die auf eine Substanz oder deren Entzug zurückzuführen sind, insbesondere Kopfschmerzen, die auf Übergebrauch, Dauereinnahme, Missbrauch oder Entzug von Suchtmitteln, Drogen oder Medikamente zurückzuführen sind, Kopfschmerzen, die auf eine Infektion oder auf eine Störung der Homöostase zurückzuführen sind, Kopfschmerzen oder Gesichtsschmerzen, die auf Erkrankungen des Schädels sowie von Hals, Augen, Ohren, Nase, Nebenhöhlen, Zähnen, Mund oder anderen Gesichts- oder Schädelstrukturen zurückzuführen sind, oder Schmerzen, die auf psychiatrische Störungen zurückzuführen sind; oder kraniale Neuralgien, zentraler und primärer Gesichtsschmerz und andere Kopfschmerzen, umfassend bspw. kraniale Neuralgien, zentrale Ursachen von Gesichtsschmerzen, andere Kopfschmerzen sowie kraniale Neuralgien, zentraler und primärer Gesichtsschmerz, insbesondere Trigeminus-Neuralgie, und dass die chronischen SchmerzErkrankungen ausgewählt werden aus Erkrankungen, umfassend Zoster- und postzosterische Neuralgien, chronische neuropathische Schmerzen (periphere Nervenläsionen, Wurzelläsionen) (chronische) Phantom-Schmerzen, chronische posttraumatische Schmerzen, chronische postoperative Schmerzen, chronisch auftretenden Menorrhagien und Ischialgien, (chronische) LWS-Syndrome (radikulär und pseudoradikulär), chronische Schmerzen nach Bandscheibenvorfall, chronische Schmerzen bei Wirbelfrakturen, chronische Schmerzerkrankungen aufgrund von degenerativen Prozessen der Wirbelsäule, der Gelenke sowie des skeletto-muskulären Apparats, chronische Schmerzen aufgrund von (therapieresistenten) Sportverletzungen oder Tendopathien, komplexe regionale chronisch auftretende Schmerzsyndrome (CRPSI, CRPSII), chronische Schmerzen bei arbeitsbedingter Fehlhaltung (Kassiererschmerz, Computer-Maus etc.), chronische Schmerzen aufgrund von Intensionstendopathien, chronisch gewordene Tumor-/Krebsschmerzen, chronisch gewordene Schmerzen bei Tumormetastasen der Wirbelsäule, andere therapieresistente chronisch gewordene Tumorschmerzen, (z.B. Pancreaskarzinom, Ovarialkarzinom), chronische Schmerzen aufgrund von Angina pectoris ohne Coronarsklerose, andere chronische cardiale Schmerzen ohne organische Ursache, chronische Schmerzen aufgrund von Schulter-Arm-Syndrom (radikulär und pseudoradikulär), Nackenschmerzen, akuter Herpes zoster, postzosterische Neuralgie, Intercostalneuralgie, chronische viszerale Schmerzen, chronische Schmerzformen neuroimmuner Genese, chronische urogenitale Schmerzen, chronische Schmerzen aufgrund von Arthritis, Arthrose, arterieller Verschlusskrankheit, oder Morbus Raynaud, oder chronische Schmerzen aufgrund von sogenannten Non-pain Syndromes, umfassend Neurodermitis, (verzögerte) Wundheilung, Tinnitus, Hörsturz, Schwindel, oder Sweating.

10. Pharmazeutische Zusammensetzung zur Verwendung in der Therapie oder Prophylaxe von chronischen Schmerz- oder Kopfschmerzerkrankungen durch Infiltrations-Anästhesie, enthaltend Lidocain oder oder ein 3-OH oder 4-OH-Derivat davon in einer Konzentration von 0,05 Gew.-% bis 0,2 Gew.-%.

## Claims

1. Lidocaine or a 3-OH or 4-OH derivative thereof in a concentration of from 0.05 wt.% to 0.2 wt.% for use in the therapy or prophylaxis of chronic pain or headache disorders by infiltration anaesthesia.

2. Lidocaine or a 3-OH or 4-OH derivative thereof according to claim 1 for use according to claim 1, **characterised in that** the chronic headache disorder is a primary headache disorder, preferably migraine.

3. Lidocaine or a 3-OH or 4-OH derivative thereof according to either claim 1 or claim 2 for use according to claim 1 or 2, **characterised in that** the low-dose lidocaine or a 3-OH or 4-OH derivative thereof is used in a concentration of from 0.05 wt.% to 0.1 wt.%, in a concentration of from 0.05 wt.% to 0.09 wt.% or in a concentration of from 0.05 wt.% to 0.08 wt.%.

4. Lidocaine or a 3-OH or 4-OH derivative thereof according to any one of claims 1 to 3 for use according to any one of claims 1 to 3, **characterised in that** the infiltration anaesthesia is carried out by injection in the region of the cervical spine, the neck muscles, the craniocervical junction (nape of the neck), the upper, middle or lower thoracic spine, for example in the region of thoracic segments/sympathetic trunk ganglia T1, T2, T3, T4, T5, T6, T7, T8, T9, T10, T11 or T12 (also called Th1, Th2, Th3, Th4, Th5, Th6, Th7, Th8, Th9, Th10, Th11 or Th12), in the region of lumbar segments L1, L2, L3, L4 or L5, at the base of the skull (ventral rami of C1) as well as the ventral rami of spinal nerves C2, C3, C4, C5, C6, C7, C8, in the region of the cerebral arteries, the nerve cords in those regions, in the region of the neck muscles, of thoracic segments T1 to T3, of the craniocervical junction (nape of the neck), in the region of the ganglion cervicale superius and stellatum, of the splenus capitis, of the semispinalis capitis, of the longissimus capitis, as well as at the base of the skull (ventral rami of C1) or the ventral rami of spinal nerves C2 and C3.

5. Lidocaine or a 3-OH or 4-OH derivative thereof according to any one of claims 1 to 4 for use according to any one of claims 1 to 4, **characterised in that** the low-dose lidocaine or a 3-OH or 4-OH derivative thereof additionally comprises at least one penetration agent or penetration enhancer.

6. Lidocaine or a 3-OH or 4-OH derivative thereof according to claim 5 for use according to claim 5, **characterised in that** the penetration agent or penetration enhancer is selected from benzyl alcohol, (2-(2-ethoxy)ethanol, propylene glycol, methylpyrrolidone or dimethyl sulfoxide (DMSO).

7. Lidocaine or a 3-OH or 4-OH derivative thereof according to any one of claims 1 to 6 for use according to any one of claims 1 to 6, **characterised in that** the pharmaceutical composition is administered once or several times per day, per week or per month.

8. Lidocaine or a 3-OH or 4-OH derivative thereof according to any one of claims 1 to 7 for use according to any one of claims 1 to 7, **characterised in that** the pharmaceutical composition is administered from 2 to 5 times, from 5 to 10 times, from 10 to 20 times or from 20 to 40 times per day, per week, per month or per year.

9. Lidocaine or a 3-OH or 4-OH derivative thereof according to any one of claims 1 to 8 for use according to any one of claims 1 to 8, **characterised in that** the chronic headache disorders are selected from the group comprising primary headache disorders, including migraine, migraine with and without aura, retinal migraine, chronic migraine, tension-type headache, cluster headache, other trigemino-autonomic headache disorders and other primary (optionally juvenile) headaches; or secondary headache disorders, including headaches attributable to head and/or cervical spine (CS) trauma, headaches attributable to vascular disorders in the region of the head or neck, headaches attributable to non-vascular intracranial disorders, headaches attributable to a substance or the withdrawal thereof, in particular headaches attributable to overuse, long-term intake, abuse or withdrawal of addictive substances, drugs or medicaments, headaches attributable to an infection or to a disturbance of homeostasis, headaches or facial pain attributable to disorders of the skull and of the neck, eyes, ears, nose, sinuses, teeth, mouth or other facial or skull structures, or pain attributable to psychiatric disorders; or cranial neuralgia, central and primary facial pain and other headaches, including, for example, cranial neuralgia, central causes of facial pain, other headaches as well as cranial neuralgia, central and primary facial pain, in particular trigeminal neuralgia, and **in that** the chronic pain disorders are selected from disorders including herpetic and post-herpetic neuralgia, chronic neuropathic pain (peripheral nerve lesions, root lesions), (chronic) phantom pain, chronic post-traumatic pain, chronic post-operative pain, chronic menorrhagia and ischialgia, (chronic) lumbar spine syndrome (radicular and pseudoradicular), chronic pain following a slipped disc, chronic pain in the case of vertebral fractures, chronic pain disorders due to degenerative processes of the spinal column, of the joints and of the musculoskeletal apparatus, chronic pain due to (therapy-resistant) sports injuries or tendinopathies, complex regional pain syndrome (CRPSI, CRPSII), chronic pain in the case of work-related bad posture (cashier's pain, computer mouse, etc.), chronic pain due to intensional tendinopathies, tumour/cancer pain that has become chronic, pain that has become chronic in the case of tumour metastases of the spinal column, other therapy-resistant tumour pain that has become chronic (e.g. pancreatic carcinoma, ovarian carcinoma), chronic pain due to Angina pectoris without coronary sclerosis, other chronic cardiac pain without an organic cause, chronic pain due to shoulder-arm syndrome (radicular and pseudoradicular), neck pain, acute Herpes zoster, post-herpetic neuralgia, intercostal neuralgia, chronic visceral pain, chronic forms of pain of neuroimmune origin, chronic urogenital pain, chronic pain due to arthritis, arthrosis, arterial obstructive disease, or Raynaud's disease, or chronic pain due to so-called non-pain syndromes, including neurodermatitis, (delayed) wound healing, tinnitus, hearing loss, dizziness or sweating.

10. Pharmaceutical composition for use in the therapy or prophylaxis of chronic pain or headache disorders by infiltration anaesthesia, comprising lidocaine or a 3-OH or 4-OH derivative thereof in a concentration of from 0.05 wt.% to 0.2 wt.%.

## Revendications

1. Lidocaïne ou un dérivé 3-OH ou 4-OH de celle-ci, en une concentration de 0,05 % en poids à 0,2 % en poids pour son utilisation dans le traitement ou la prophylaxie de douleurs ou céphalées chroniques par anesthésie par infiltration.

2. Lidocaïne ou un dérivé 3-OH ou 4-OH de celle-ci, selon la revendication 1, pour son utilisation selon la revendication 1, **caractérisée en ce que** la céphalée chronique est une céphalée primaire, de préférence une migraine.

3. Lidocaïne ou un dérivé 3-OH ou 4-OH de celle-ci, selon l'une des revendications 1 ou 2, pour son utilisation selon la revendication 1 ou 2, **caractérisée en ce que** la lidocaïne faiblement dosée ou un dérivé 3-OH ou 4-OH de celle-ci est utilisé(e) en une concentration de 0,05 % en poids à 0,1 % en poids, en une concentration de 0,05 % en poids à 0,09 % en poids ou en une concentration de 0,05 % en poids à 0,08 % en poids.

4. Lidocaïne ou un dérivé 3-OH ou 4-OH de celle-ci, selon l'une des revendications 1 à 3, pour son utilisation selon l'une des revendications 1 à 3, **caractérisée en ce que** l'anesthésie par infiltration est réalisée par injection dans la région de la colonne cervicale, des muscles du cou, de la transition crânio-cervicale (nuque), de la colonne thoracique supérieure, médiane ou inférieure, par exemple dans la région des segments thoraciques/ganglions de la chaîne sympathique T1, T2, T3, T4, T5, T6, T7, T8, T9, T10, T11 ou T12 (également nommés Th1, Th2, Th3, Th4, Th5, Th6, Th7, Th8, Th9, Th10, Th11 ou Th12), dans la zone des segments lombaires L1, L2, L3, L4 ou L5, à la base du crâne (branches ventrales de C1) et les branches ventrales des nerfs spinaux C2, C3, C4, C5, C6, C7, C8, dans la région des artères cérébrales, des faisceaux nerveux dans ces régions, dans la région des muscles du cou, des segments thoraciques T1 à T3, de la transition crânio-cervicale (nuque), dans la région du ganglion cervical supérieur et du ganglion stellaire, du muscle splénius de la tête, du muscle semi-spinal de la tête, du muscle longissimus de la tête et à la base du crâne (branche ventrale de C1) ou de la branche ventrale des nerfs spinaux C2 et C3.

5. Lidocaïne ou un dérivé 3-OH ou 4-OH de celle-ci, selon l'une des revendications 1 à 4, pour son utilisation selon l'une des revendications 1 à 4, **caractérisée en ce que** la lidocaïne faiblement dosée ou un dérivé 3-OH ou 4-OH de celle-ci contient en outre au moins un agent de pénétration ou un agent de renforcement de pénétration.

6. Lidocaïne ou un dérivé 3-OH ou 4-OH de celle-ci, selon la revendication 5 pour son utilisation selon la revendication 5, **caractérisée en ce que** l'agent de pénétration ou l'agent de renforcement de pénétration est choisi parmi l'alcool benzylique, le (2-(2-éthoxy)éthanol), le propylène glycol, la méthylpyrrolidone ou le diméthylsulfoxyde (DMSO).

7. Lidocaïne ou un dérivé 3-OH ou 4-OH de celle-ci, selon l'une des revendications 1 à 6, pour son utilisation selon l'une des revendications 1 à 6, **caractérisée en ce que** la composition pharmaceutique est administrée une fois ou plusieurs fois par jour, par semaine ou par mois.

8. Lidocaïne ou un dérivé 3-OH ou 4-OH de celle-ci, selon l'une des revendications 1 à 7, pour son utilisation selon l'une des revendications 1 à 7, **caractérisée en ce que** la composition pharmaceutique est administrée 2 à 5 fois, 5 à 10 fois, 10 à 20 fois ou 20 à 40 fois par jour, par semaine, par mois ou par an.

9. Lidocaïne ou un dérivé 3-OH ou 4-OH de celle-ci, selon l'une des revendications 1 à 8, pour son utilisation selon l'une des revendications 1 à 8, **caractérisée en ce que** les céphalées chroniques sont choisies dans le groupe comprenant les céphalées primaires comprenant la migraine, la migraine avec ou sans aura, la migraine rétinienne, la migraine chronique, les céphalées de type tension, les céphalées en grappe, d'autres céphalées trigéminales autonomes et autres céphalées primaires (éventuellement juvéniles) ; ou les céphalées secondaires comprenant les céphalées qui sont imputables à un traumatisme de la colonne au niveau de la tête et/ou cervicale, les céphalées qui sont imputables à des troubles vasculaires dans la région de la tête ou du cou, les céphalées qui sont imputables à des troubles intracrâniens non vasculaires, les céphalées qui sont imputables à une substance ou leur sevrage, en particulier les céphalées qui sont imputables à une surconsommation, une prise permanente, un abus ou un sevrage de substances addictives, de drogues ou de médicaments, les céphalées qui sont imputables à une infection ou à un trouble de l'homéostasie, les céphalées ou les douleurs faciales qui sont imputables à des maladies du crâne et du cou, des yeux, des oreilles, du nez, des sinus, des dents, de la bouche et d'autres structures crânio-faciales, ou les douleurs qui sont imputables à des troubles psychiatriques ; ou les névralgies crâniennes, les douleurs faciales centrales et primaires et autres céphalées, comprenant par exemple les névralgies crâniennes, les causes centrales des douleurs faciales, d'autres céphalées et névralgies crâniennes, les douleurs faciales centrales et primaires, en particulier les névralgies trigéminales, et **en ce que** les céphalées chroniques sont choisies parmi les maladies comprenant les névralgies liées au zona et les névralgies post-zostériennes, les douleurs neuropathiques chroniques (lésions nerveuses périphériques, lésions radiculaires), les douleurs fantômes (chroniques), les douleurs post-traumatiques chroniques, les douleurs postopératoires chroniques, les ménorragies et sciatalgies survenant de manière chronique, le syndrome LWS (chronique) (radiculaire et pseudo-radiculaire), les douleurs chroniques consécutives à une hernie discale, les douleurs chroniques dans les fractures de la colonne vertébrale, les douleurs chroniques dues à des processus dégénératifs de la colonne vertébrale, des articulations et de l'appareil musculo-squelettique, les douleurs chroniques dues à des blessures sportives (résistantes au traitement) ou les tendinopathies, les syndromes douloureux complexes régionaux survenant de façon chronique (CRPSI, CRPSII), les douleurs chroniques liées à une mauvaise posture dans le cadre professionnel (douleurs des caissières, douleurs liées à la souris d'ordinateur, etc.), les douleurs chroniques dues à des tendinopathies intensionnelles, les douleurs tumorales/cancéreuses devenues chroniques, les douleurs devenues chroniques dans le cas de métastases tumorales de la colonne vertébrale, d'autres douleurs tumorales devenues chroniques, résistantes au traitement (par exemple cancer du pancréas, cancer de l'ovaire), les douleurs chroniques due à l'angine de poitrine sans sclérose coronarienne, d'autres douleurs cardiaques chroniques sans cause organique, les douleurs chroniques dues au syndrome épaule-bras (radiculaire et pseudoradiculaire), les douleurs de la nuque, le zona aigu, les névralgies post-zostériennes, les névralgies intercostales, les douleurs viscérales chroniques, les formes douloureuses chroniques d'origine neuro-immune, les douleurs urogénitales chroniques, les douleurs chroniques dues à l'arthrite, l'arthrose, les maladies artérielles oblitérantes ou la maladie de Raynaud ou les douleurs chroniques dues à des syndromes dits non douloureux comprenant la neurodermite, la cicatrisation des plaies (retardée), les bourdonnements d'oreille, les surdités brusques, les vertiges ou la transpiration.

10. Composition pharmaceutique pour son utilisation dans le traitement ou la prophylaxie de douleurs ou céphalées chroniques par anesthésie par infiltration, contenant de la lidocaïne ou un dérivé 3-OH ou 4-OH de celle-ci, en une concentration de 0,05 % en poids à 0,2 % en poids.
